# EUROPEAN PATENT APPLICATION

(11) **EP 4 821 508 A1**
(43) Date of publication of application: **14.10.2026**
(21) Application number: 24900614.9
(22) Date of filing: 03.12.2024
(51) Int. Cl.: A61K 45/00, A61K 31/18, A61K 31/4035, A61K 31/426, A61K 31/519, A61P 13/12

(54) **PROPHYLACTIC OR THERAPEUTIC AGENT FOR POLYCYSTIC KIDNEY DISEASE**

(30) Priority: 04.12.2023 JP 2023204867
(71) Applicant: Yamaguchi University, Yamaguchi 753-8511 (JP); NAGASAKI UNIVERSITY, Nagasaki 852-8521 (JP); Hiroshima University, Higashihiroshima-shi Hiroshima 739-8511 (JP)
(72) Inventor: MIYAMOTO Tatsuo, Ube-shi, Yamaguchi 755-8505 (JP); ITABASHI Takeshi, Ube-shi, Yamaguchi 755-8505 (JP); MORITA Tomoka, Ube-shi, Yamaguchi 755-8505 (JP); SUZUKI Keiji, Nagasaki-shi, Nagasaki 852-8521 (JP); YAMAMOTO Takashi, Higashihiroshima-shi, Hiroshima 739-8511 (JP); HOSOBA Kosuke, Higashihiroshima-shi, Hiroshima 739-8511 (JP)
(74) Representative: Hasegawa, Kan
(86) International application number: PCT/JP2024/042728
(87) International publication number: WO 2025/121320

(57) **Abstract**

An object of the present invention is to provide a novel prophylactic or therapeutic agent for kidney disease.

A prophylactic or therapeutic agent for polycystic kidney disease is provided, including, as an active ingredient, a compound that has an activity of promoting the intermolecular interaction between oxysterol-binding protein-related protein 3 (ORP3) and vesicle-associated membrane protein-associated protein A (VAP-A) in cells and has an activity of increasing cholesterol in primary cilia of cells, or a pharmaceutically acceptable salt thereof.

## Description

### Technical Field

The present invention relates to a prophylactic or therapeutic agent for polycystic kidney disease.

### Background Art

Autosomal dominant polycystic kidney disease (ADPKD) is a hereditary disease caused by mutations in the polycystin complex (polycystin 1 and polycystin 2 form a tetramer in a 3:1 ratio), which functions as a mechanosensitive calcium ion channel localized in the primary cilia of renal epithelial cells. ADPKD is designated as an intractable disease (designated intractable disease number 67), and the number of patients in Japan was estimated to be 31,000 as of 1994. Approximately half of the patients progress to end-stage renal failure by the age of 70, and complications such as hepatic cysts and cerebral aneurysms are also observed. No fundamental treatment for this disease has been established. As a treatment for slowing disease progression, tolvaptan, a vasopressin V2 receptor antagonist, has been approved for insurance coverage and is used (see Patent literature 1). However, administration of this drug may cause dehydration and hypernatremia due to rapid excretion of water or the like. In particular, there have been concerns that its efficacy and safety have not been established in patients with impaired renal function or in pediatric patients (see Non-patent literature 1).

As other therapeutic agents for ADPKD, octreotide (a somatostatin analog), a mTOR (mammalian target of rapamycin) inhibitor, venglustat (a glucosylceramide synthase inhibitor), and the like have been studied (see Non-patent literature 2). However, like tolvaptan, these are also therapeutic agents that target signal transduction pathways.

Meanwhile, the present inventors have conducted studies on the promotion of cholesterol transport to primary cilia, and have disclosed that oxysterol-binding protein (OSBP)-related protein 3 (ORP3), which is localized at the primary ciliary pocket, is responsible for transporting cholesterol from peroxisomes to primary cilia (see Non-patent literature 3). However, the relationship between cholesterol transport to primary cilia and polycystic kidney disease has not been known to date.

### Citation List

### Patent Literature

Patent Literature 1: U.S. Patent No. 5,258,510

### Non-Patent Literature

Non-Patent Literature 1: Kazuo Kamohara, Evidence-Based Clinical Practice Guidelines for Polycystic Kidney Disease (PKD), 2017, Tokyo Igakusha.
Non-Patent Literature 2: Shigeo Horie, New strategy for the treatment of autosomal dominant polycystic kidney disease, Japanese Journal of Nephrology, 2015; 57(1): 254-261
Non-Patent Literature 3: Miyamoto et al., The EMBO Journal (2020) 39: e103499

### Summary of the Invention

### Technical Problem

An object of the present invention is to provide a novel prophylactic or therapeutic agent for polycystic kidney disease.

### Means for Solving the Problem

In normal renal tubular epithelial cells, renal cilia sense the flow rate of urine, and the thickness (diameter) of renal tubules is regulated by a complex of polycystin 1 involved in the proliferation and differentiation of renal tubular cells and the sensing of urine flow on the renal cilia, and polycystin 2, which is a calcium channel. In this context, it is hypothesized that dysfunction resulting from mutations or depletion of the polycystin complex in primary cilia leads to the constitutive activation of the intracellular cAMP-PKA pathway; this, in turn, drives increased cell proliferation and enhanced secretion, such as that of chloride ions, into the epithelial lumen, ultimately causing the dilation of renal tubules and collecting ducts, and the subsequent formation of cysts.

The present inventors have previously found that induction of organelle tethering between primary cilia and peroxisomes using an FRB-FKBP system (a rapamycin-dependent heteromolecular tethering system) increases cholesterol in primary cilia in an ORP3/vesicle-associated membrane protein-associated protein A (VAP-A)-dependent manner. Based on this finding, the present inventors hypothesized that, in patients with polycystic kidney disease in whom transport of the polycystin complex to primary cilia is impaired, promotion of cholesterol transport to primary cilia through activation of the ORP3/VAP-A system would improve localization of the polycystin complex to primary cilia and suppress progression of the disease through recovery of renal function. Accordingly, the present inventors have searched for compounds that increase cholesterol transport to primary cilia and contribute to improvement of the pathological condition of polycystic kidney disease. As a result, the present inventors have found compounds that promote the intermolecular interaction between ORP3 and VAP-A, thereby enhancing transport of polycystin 1 and polycystin 2 to primary cilia and improving the pathological condition of polycystic kidney disease, and thus completed the present invention.

Specifically, the present invention provides the following.
[1] A prophylactic or therapeutic agent for polycystic kidney disease, including, as an active ingredient, a compound having an activity of promoting an intermolecular interaction between oxysterol-binding protein-related protein 3 (ORP3) and vesicle-associated membrane protein-associated protein A (VAP-A) in a cell and having an activity of increasing cholesterol in primary cilia of a cell, or a pharmaceutically acceptable salt thereof.
[2] The prophylactic or therapeutic agent for polycystic kidney disease according to the above [1], in which the compound having the activity of promoting the intermolecular interaction and having the activity of increasing cholesterol in primary cilia of a cell is a compound including:
   a 4-amino-1-phenylpyrazolopyrimidine skeleton;
   a 5-(sulfamoyl)benzamide skeleton;
   an N-thiazolylbenzamide skeleton; or
   an N-benzylsuccinimide skeleton.
[3] The prophylactic or therapeutic agent for polycystic kidney disease according to the above [1], in which the compound having the activity of promoting the intermolecular interaction and having the activity of increasing cholesterol in primary cilia of a cell is a 4-amino-1-phenylpyrazolopyrimidine compound represented by a general formula (I): 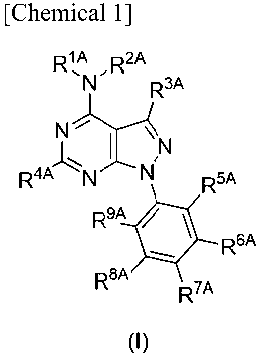
   (wherein R^{1A} and R^{2A} are the same or different and each represent:
      a hydrogen atom;
      a C1-6 alkyl group optionally having a substituent;
      a C1-6 alkoxy group optionally having a substituent;
      a C2-6 alkenyl group optionally having a substituent;
      a C2-6 alkynyl group optionally having a substituent; or
      a phenyl group optionally having a substituent,
   R^{3A} and R^{4A} are the same or different and each represent:
      a hydrogen atom;
      a halogen atom;
      a nitro group;
      a C1-6 alkyl group optionally having a substituent;
      a C1-6 alkoxy group optionally having a substituent; or
      a phenyl group optionally having a substituent, and
   R^{5A}, R^{6A}, R^{7A}, R^{8A}, and R^{9A} are the same or different and each represent:
      a hydrogen atom;
      a halogen atom;
      a nitro group;
      a C1-6 alkyl group optionally having a substituent; or
      a C1-6 alkoxy group optionally having a substituent); or a pharmaceutically acceptable salt thereof.
[4] The prophylactic or therapeutic agent for polycystic kidney disease according to the above [1], in which the compound having the activity of promoting the intermolecular interaction and having the activity of increasing cholesterol in primary cilia of a cell is:
   a compound represented by a following formula (I-1) (4-methoxypropylamino-1-(2,4-dimethylphenyl)-1H-pyrazolo[3,4-d]pyrimidine);
   a compound represented by a following formula (I-2) (4-(4-fluorobenzylamino)-1-(3-methylphenyl)-1H-pyrazolo[3,4-d]pyrimidine);
   a compound represented by a following formula (I-3) (4-(3,5-dimethylphenyl)amino-1-(4-methoxyphenyl)-1H-pyrazolo[3,4-d]pyrimidine);
   a compound represented by a following formula (I-4) (2-(4-chlorophenyl)ethylamino-1-(3-methylphenyl)-1H-pyrazolo[3,4-d]pyrimidine);
   a compound represented by a following formula (I-5) (2-(1-cyclohexenyl)ethylamino-1-(3-methylphenyl)-1H-pyrazolo[3,4-d]pyrimidine); or
   a compound represented by a following formula (I-6) (4-hydroxypropylamino-1-(2,4-dimethylphenyl)-1H-pyrazolo[3,4-d]pyrimidine). 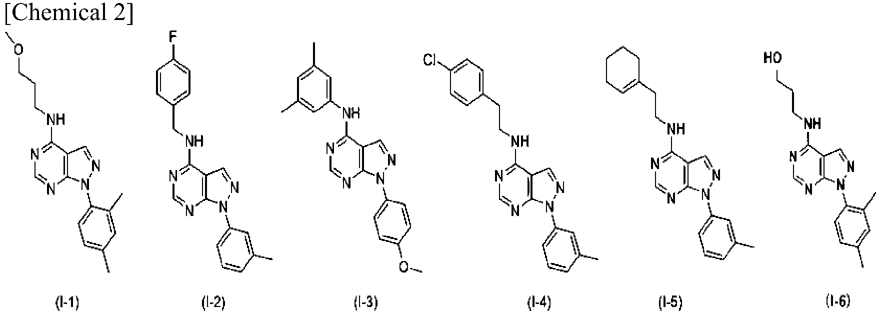
[5] The prophylactic or therapeutic agent for polycystic kidney disease according to the above [1], in which the compound having the activity of promoting the intermolecular interaction and having the activity of increasing cholesterol in primary cilia of a cell is a 5-(sulfamoyl)benzamide compound represented by a general formula (II). 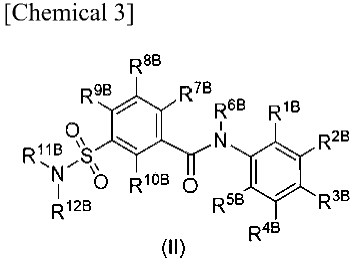
   (wherein R^{1B}, R^{2B}, R^{3B}, R^{4B}, R^{5B}, R^{7B}, R^{8B}, R^{9B}, and R^{10B} are the same or different and each represent:
      a hydrogen atom;
      a halogen atom;
      a nitro group;
      a C1-6 alkyl group optionally having a substituent;
      a C1-6 alkoxy group optionally having a substituent; or
      a phenyl group optionally having a substituent,
   R^{6B} represents:
      a hydrogen atom; or
      a C1-6 alkyl group optionally having a substituent, and
   R^{11B} and R^{12B} are the same or different and each represent:
      a hydrogen atom;
      a C1-6 alkyl group optionally having a substituent;
      a C1-6 alkoxy group optionally having a substituent;
      a C3-8 cycloalkyl group optionally having a substituent;
      a C3-6 cycloalkoxy group optionally having a substituent; or
      a phenyl group optionally having a substituent)
[6] The prophylactic or therapeutic agent for polycystic kidney disease according to the above [1], in which the compound having the activity of promoting the intermolecular interaction and having the activity of increasing cholesterol in primary cilia of a cell is a compound represented by a following formula (II-1) or a compound represented by a following formula (II-2). 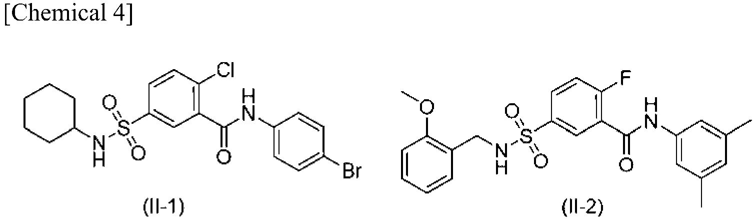
[7] The prophylactic or therapeutic agent for polycystic kidney disease according to the above [1], in which the compound having the activity of promoting the intermolecular interaction and having the activity of increasing cholesterol in primary cilia of a cell is an N-thiazolylbenzamide compound represented by a general formula (III). 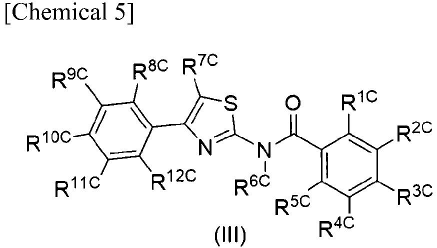
   (wherein R^{1C}, R^{2C}, R^{3C}, R^{4C}, R^{5C}, R^{8C}, R^{9C}, R^{10C}, R^{11C}, and R^{12C} are the same or different and each represent:
      a hydrogen atom;
      a halogen atom;
      a nitro group;
      a C1-6 alkyl group optionally having a substituent;
      a C1-6 alkoxy group optionally having a substituent; or
      a phenyl group optionally having a substituent,
   R^{6C} represents:
      a hydrogen atom; or
      a C1-6 alkyl group optionally having a substituent, and
   R^{7C} represents:
      a hydrogen atom;
      a halogen atom;
      a C1-6 alkyl group optionally having a substituent;
      a C1-6 alkoxy group optionally having a substituent; or
      a phenyl group optionally having a substituent)
[8] The prophylactic or therapeutic agent for polycystic kidney disease according to the above [1], in which the compound having the activity of promoting the intermolecular interaction and having the activity of increasing cholesterol in primary cilia of a cell is a compound represented by a following formula (III-1). 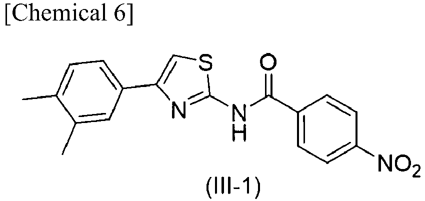
[9] The prophylactic or therapeutic agent for polycystic kidney disease according to the above [1], in which the compound having the activity of promoting the intermolecular interaction and having the activity of increasing cholesterol in primary cilia of a cell is an N-benzylsuccinimide compound represented by a general formula (IV). 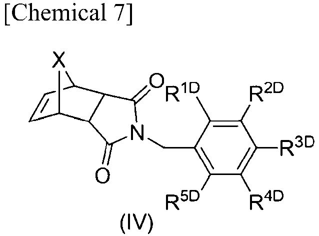
   (wherein R^{1D}, R^{2D}, R^{3D}, R^{4D}, and R^{5D} are the same or different and each represent:
      a hydrogen atom; or
      a C1-6 alkyl group optionally having a substituent, and
   X represents an oxygen atom or CH₂)
[10] The prophylactic or therapeutic agent for polycystic kidney disease according to the above [1], in which the compound having the activity of promoting the intermolecular interaction and having the activity of increasing cholesterol in primary cilia of a cell is a compound represented by a following formula (IV-1). 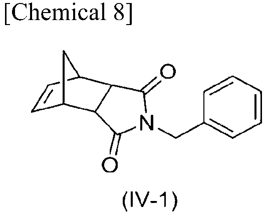
[11] The prophylactic or therapeutic agent for polycystic kidney disease according to the above [1], for administration to a patient having a gene mutation in PKD1 and/or PKD2.

### Effects of the Invention

According to the present invention, a novel prophylactic or therapeutic agent for polycystic kidney disease, having an action mechanism different from that of existing drugs, can be provided.

### Brief Description of Drawings

FIG. 1 shows the results of measuring luminescence of hit compounds obtained from a compound library using a screening platform constructed by introducing a split-luciferase system targeting an intermolecular interaction between ORP3 and VAPA in Example 1.
FIG. 2A shows the results of observing primary ciliary cholesterol upon treatment with a compound A, using a confocal laser microscope, in a primary ciliary cholesterol delivery activity assay in Example 2.
FIG. 2B shows the results of measuring fluorescence intensity of Filipin III in primary cilia upon treatment with the compound A in the primary ciliary cholesterol delivery activity assay in Example 2.
FIG. 3A shows the results of observing primary ciliary cholesterol upon treatment with compounds A, C, and D, using a confocal laser microscope, in the primary ciliary cholesterol delivery activity assay in Example 2.
FIG. 3B shows the results of measuring the fluorescence intensity of Filipin III in primary cilia upon treatment with the compounds A, C, and D in the primary ciliary cholesterol delivery activity assay in Example 2.
FIG. 4A shows the results of observing primary ciliary cholesterol upon treatment with compounds A, AB, AF, AL, AP, R, and V, using a confocal laser microscope, in the primary ciliary cholesterol delivery activity assay in Example 2.
FIG. 4B shows the results of measuring the fluorescence intensity of Filipin III in primary cilia upon treatment with the compounds A, AB, AF, AL, AP, R, and V in the primary ciliary cholesterol delivery activity assay in Example 2.
FIG. 5A shows the results of observing primary ciliary cholesterol upon treatment with compounds A and I, using a confocal laser microscope, in the primary ciliary cholesterol delivery activity assay in Example 2.
FIG. 5B shows the results of measuring the fluorescence intensity of Filipin III in primary cilia upon treatment with the compounds A and I in the primary ciliary cholesterol delivery activity assay in Example 2.
FIG. 6A shows the results of observing primary ciliary cholesterol upon treatment with a compound C, using a confocal laser microscope, in a recovery assay of primary ciliary localization of a polycystin 1 missense protein in Example 3.
FIG. 6B shows the results of measuring fluorescence intensity of polycystin 1 in primary cilia upon treatment with the compound C in the recovery assay of primary ciliary localization of the polycystin 1 missense protein in Example 3.
FIG. 7A shows the results of observing cysts formed by collecting duct-derived cells of a PC1 mutant mouse upon treatment with compounds A, C, and R by staining with phalloidin (staining for F-actin cytoskeleton) and DAPI (staining for DNA), using a confocal laser microscope, in a tubule diameter enlargement inhibitory assay using a cyst culture system in Example 4.
FIG. 7B shows the results of observing cysts formed by collecting duct-derived cells of a PC2 mutant mouse upon treatment with the compounds A, C, and R by staining with phalloidin and DAPI, using a confocal laser microscope, in the tubule diameter enlargement inhibitory assay using the cyst culture system in Example 4.
FIG. 7C shows the results of determining an inner diameter/outer diameter ratio in merged images of cysts formed upon treatment with the compounds A, C, and R in the tubule diameter enlargement inhibitory assay using the cyst culture system in Example 4.
FIG. 8A shows the results of observing cysts formed by collecting duct-derived cells of the PC2 mutant mouse upon treatment with compounds A, G, and R, and tolvaptan as an existing drug, by staining with phalloidin and DAPI, using a confocal laser microscope, in the tubule diameter enlargement inhibitory assay using the cyst culture system in Example 5.
FIG. 8B shows the results of determining the inner diameter/outer diameter ratio in merged images of cysts formed by collecting duct-derived cells of the PC2 mutant mouse upon treatment with the compounds A, G, and R, and tolvaptan as an existing drug in the tubule diameter enlargement inhibitory assay using the cyst culture system in Example 5.
FIG. 9A shows the results of observing cysts formed by collecting duct-derived cells of the PC2 mutant mouse upon treatment with the compounds A and G, and a compound G-OH, which is a derivative of the compound G, by staining with phalloidin and DAPI, using a confocal laser microscope, in the tubule diameter enlargement inhibitory assay using the cyst culture system in Example 5.
FIG. 9B shows the results of determining the inner diameter/outer diameter ratio in merged images of cysts formed by collecting duct-derived cells of the PC2 mutant mouse upon treatment with the compounds A and G, and the compound G-OH, which is a derivative of the compound G, in the tubule diameter enlargement inhibitory assay using the cyst culture system in Example 5.
FIG. 10A shows the results of observing cysts formed by collecting duct-derived cells of the PC2 mutant mouse upon treatment with compounds A, G, B, Q, K, and J by staining with phalloidin and DAPI, using a confocal laser microscope, in the tubule diameter enlargement inhibitory assay using the cyst culture system in Example 6.
FIG. 10B shows the results of determining the inner diameter/outer diameter ratio in merged images of cysts formed by collecting duct-derived cells of the PC2 mutant mouse upon treatment with the compounds A, G, B, Q, K, and J in the tubule diameter enlargement inhibitory assay using the cyst culture system in Example 6.
FIG. 11A shows the results of observing cysts formed by collecting duct-derived cells of the PC2 mutant mouse upon treatment with compounds A and I by staining with phalloidin and DAPI, using a confocal laser microscope, in the tubule diameter enlargement inhibitory assay using the cyst culture system in Example 6.
FIG. 11B shows the results of determining the inner diameter/outer diameter ratio in merged images of cysts formed by collecting duct-derived cells of the PC2 mutant mouse upon treatment with the compounds A and I in the tubule diameter enlargement inhibitory assay using the cyst culture system in Example 6.
FIG. 12A shows the results of observing polycystin 2 protein in primary cilia of human induced pluripotent stem cells derived from a patient with polycystic kidney disease upon treatment with the compound G-OH and tolvaptan as an existing drug, using a confocal laser microscope, in a recovery assay of primary ciliary localization of polycystin 2 protein in Example 7.
FIG. 12B shows the results of measuring the amount of polycystin 2 protein (PC2) in primary cilia of human induced pluripotent stem cells derived from a patient with polycystic kidney disease upon treatment with the compound G-OH and tolvaptan as an existing drug in the recovery assay of primary ciliary localization of polycystin 2 protein in Example 7.
FIG. 13A shows the results of observing polycystin 1 protein (PC1) in primary cilia of a feline kidney-derived epithelial cell line (CRFK cells) upon treatment with the compound G, using a confocal laser microscope, in a PC1 increasing activity assay in primary cilia in Example 8.
FIG. 13B shows the results of measuring the amount of polycystin 1 protein (PC1) in primary cilia of the feline kidney-derived epithelial cell line (CRFK cells) upon treatment with the compound G in the PC1 increasing activity assay in primary cilia in Example 8.

### Modes for Carrying out the Invention

The contents described in all patent and non-patent literatures cited in the present specification are incorporated herein by reference in their entirety.

A prophylactic or therapeutic agent for polycystic kidney disease of the present invention is not particularly limited as long as it includes, as an active ingredient, a compound having an activity of promoting an intermolecular interaction between oxysterol-binding protein-related protein 3 (ORP3) and vesicle-associated membrane protein-associated protein A (VAP-A) in a cell and having an activity of increasing cholesterol in primary cilia of a cell, or a pharmaceutically acceptable salt thereof, and is hereinafter also referred to as "the present prophylactic or therapeutic agent for polycystic kidney disease".

The polycystic kidney disease in the present specification is not particularly limited, and examples thereof include autosomal dominant polycystic kidney disease (ADPKD) and autosomal recessive polycystic kidney disease (ARPKD), with ADPKD being preferable. ADPKD is caused by mutations in the polycystic kidney and hepatic disease (PKD) 1 gene on chromosome 16 and/or the PKD2 gene on chromosome 4, which encode polycystin 1 protein (PC1) and polycystin 2 protein (PC2), respectively. Further, ARPKD is caused by mutations in the PKHD1 gene on chromosome 6.

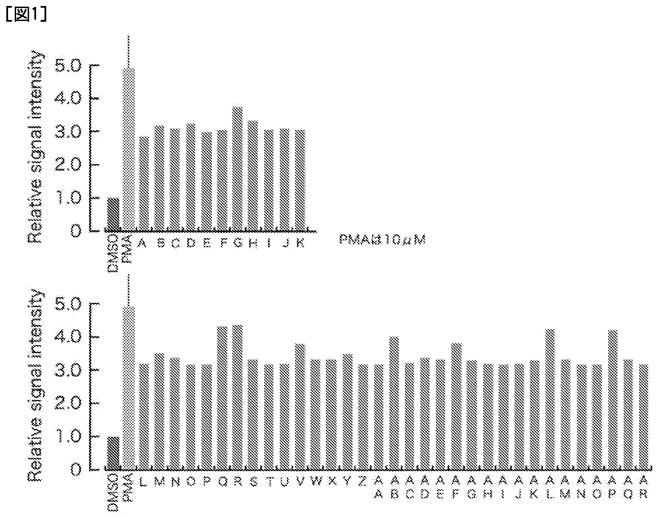

The promotion of the intermolecular interaction between oxysterol-binding protein-related protein 3 (ORP3) and vesicle-associated membrane protein-associated protein A (VAP-A) in a cell according to the present specification can be examined by known methods for analyzing protein-protein interactions, and examples thereof include split-protein systems and GST pull-down methods (Marion Weber-Boyvat et al., Experimental Cell Research, Volume 331, Issue 2, 15 February 2015, Pages 278-291 (in particular, FIG. 1); Wan Ting Saw et al., Methods, Volume 90, November 2015, Pages 68-75; Stephanie Cabantous et al., Scientific Reports, Volume 3, Article number: 2854 (2013); and JP 2003-503067 T).

As a specific example of the split-protein system, a fluorescent labeling protein is divided so as not to emit fluorescence when present alone, and one fragment of the divided fluorescent labeling protein is fused to ORP3, while the other fragment is fused to VAP-A. Next, a test compound is added to cells expressing ORP3 and VAP-A fused with the fragments of the fluorescent labeling protein, and the cells are cultured. Then, the fluorescence intensity is detected, and the activity can be evaluated by examining whether the fluorescence level upon treatment with the test compound is increased as compared with the fluorescence level in the absence of the test compound or in the presence of a compound to be compared or a positive control compound. Cells expressing ORP3 and VAP-A fused with fragments of the fluorescent labeling protein can be prepared by introducing, into cells by a known method, an expression vector including a polynucleotide encoding one fragment of the divided fluorescent labeling protein and a nucleotide encoding ORP3, and an expression vector including a polynucleotide encoding the other fragment of the divided fluorescent labeling protein and a nucleotide encoding VAP-A, and expressing them. Examples of the fluorescent label include green fluorescent protein (GFP).

Alternatively, an enzyme may be used in place of the above fluorescent label. A luminescent substrate may be added as necessary during culture, followed by detection of enzyme activity. The activity can be evaluated by examining whether the enzyme activity upon treatment with the test compound is increased as compared with the enzyme activity in the absence of the test compound or in the presence of a compound to be compared. Examples of the enzyme include luciferases. In addition, the concentration of the test compound to be added may be changed stepwise, and evaluation may be performed by detecting fluorescence intensity or enzyme activity in a concentration-dependent manner.

In the case of using the split-protein system, fluorescence intensity or enzyme activity is detected upon addition of a test compound, and the fluorescence level or enzyme activity in the absence of the test compound is defined as 1. If the measured value reaches or exceeds a predetermined threshold, the test compound can be evaluated as having an activity of promoting the intermolecular interaction between oxysterol-binding protein-related protein 3 (ORP3) and vesicle-associated membrane protein-associated protein A (VAP-A) in a cell. For example, when cells are treated with phorbol 12-myristate 13-acetate (PMA) as a positive control, the fluorescence level or enzyme activity of the treated cells is approximately 5. Therefore, when the relative level is 2.5-fold or more, more preferably 2.8-fold or more, the test compound can be evaluated as having an activity of promoting the intermolecular interaction between oxysterol-binding protein-related protein 3 (ORP3) and vesicle-associated membrane protein-associated protein A (VAP-A) in a cell.

The activity of increasing cholesterol in primary cilia of a cell according to the present specification can be evaluated by known methods. For example, cells such as retinal pigment epithelial cells are treated with a test compound at 37°C for 4 hours, followed by staining with a reagent such as a cholesterol indicator that emits fluorescence upon binding to cholesterol, such as Filipin III, or AcGFP1-D4 (a cholesterol probe in which a cholesterol-binding protein is fused to a fluorescent protein; see Miyamoto et al., Methods Mol Biol, 2022: doi: 10.1007/978-1-0716-1701-4_5.), together with a primary cilium marker, and cholesterol localized in primary cilia is observed or detected using a microscope.

Cholesterol localized in primary cilia is detected upon addition of a test compound, and the level in the absence of the test compound is defined as 1. When the relative fluorescence level is 1.2-fold or more, preferably 1.3-fold or more, and more preferably 1.4-fold or more, the test compound can be evaluated as having an activity of increasing cholesterol in primary cilia of a cell.

Further, it is preferable to confirm that the activity of increasing cholesterol in primary cilia of a cell is dependent on ORP3/VAPA. Whether the activity of increasing cholesterol in primary cilia of a cell is dependent on ORP3/VAPA can be evaluated by comparing results obtained using, as cells, wild-type retinal pigment epithelial cells with those obtained using mutant retinal pigment epithelial cells in which VAP-A or ORP3 is deficient or functionally altered. Specifically, if the activity of increasing cholesterol in primary cilia of the cells upon treatment with the test compound is observed or detected in the wild-type retinal pigment epithelial cells but not observed or detected in the mutant retinal pigment epithelial cells, the activity is evaluated as being ORP3/VAPA-dependent.

Furthermore, it is preferable that the compound of the present invention having an activity of promoting the intermolecular interaction between ORP3 and VAP-A in a cell and having an activity of increasing cholesterol in primary cilia of a cell has an activity of restoring localization of polycystin 1 to primary cilia in mouse collecting duct-derived cells (mIMCD3 cells: ATCC Cat. No. CRL-2123) expressing a polycystin 1 missense protein (a mutation in which cysteine at position 210 in the amino acid sequence of SEQ ID NO: 1 is substituted with glycine). Example of a method for confirming the above activity includes a method in which the mutant mIMCD3 cells and, if necessary, wild-type mIMCD3 cells as well, are treated with a test compound at 37°C for 4 hours, followed by staining with anti-polycystin 1 antibodies and a primary cilium marker, and polycystin 1 protein localized in primary cilia are observed or detected using a microscope.

In addition, it is preferable that the compound of the present invention having an activity of promoting the intermolecular interaction between ORP3 and VAP-A in a cell and having an activity of increasing cholesterol in primary cilia of a cell has an activity of inhibiting tubule diameter enlargement in a cyst culture system in which cyst formation is reconstructed by three-dimensional culture of renal tubular epithelial cells. Whether the compound has the activity of inhibiting tubule diameter enlargement in the cyst culture system in which cyst formation is reconstructed by three-dimensional culture of renal tubular epithelial cells can be evaluated, for example, by three-dimensionally culturing mutant mIMCD3 cells expressing a polycystin 1 missense protein, mutant mIMCD3 cells expressing a polycystin 2 missense protein, or the like, mixing the cells with a test compound and Matrigel, transferring the mixture to a chamber slide plate, and incubating the mixture at 37°C under 5% CO₂ until solidification. Thereafter, prewarmed medium is added to the solidified cell-Matrigel layer, and incubation is continued at 37°C under 5% CO₂ with medium replacement until cysts are formed. Subsequently, the cysts thus formed are fixed, sectioned, stained with phalloidin and DAPI, and then observed using a confocal laser microscope. Then, the size of the epithelial lumen formed inside the cyst in a merged image is measured as the inner diameter, and the size of the entire cyst is measured as the outer diameter to determine the inner diameter/outer diameter ratio, thereby evaluating the activity of inhibiting tubule diameter enlargement. Mutation in polycystin 1 protein or polycystin 2 protein results in reduced primary ciliary activity and leads to cyst enlargement.

The compound of the present invention having an activity of promoting the intermolecular interaction between ORP3 and VAP-A in a cell and having an activity of increasing cholesterol in primary cilia of a cell may directly act on ORP3 or VAP-A to exert the cholesterol-increasing activity, or may indirectly act on ORP3 or VAP-A through a factor capable of acting on ORP3 or VAP-A to exert the cholesterol-increasing activity. Examples of such a factor include a protein, a nucleic acid, and a lipid.

The compound to be used as an active ingredient in the present prophylactic or therapeutic agent for polycystic kidney disease is not particularly limited, and examples thereof include:
a compound including a 4-amino-1-phenylpyrazolopyrimidine skeleton (hereinafter also referred to as a "4-amino-1-phenylpyrazolopyrimidine compound" or "compound (I)");
a compound including a 5-(sulfamoyl)benzamide skeleton (hereinafter also referred to as a "5-(sulfamoyl)benzamide compound" or "compound (II)");
a compound including an N-thiazolylbenzamide skeleton (hereinafter also referred to as an "N-thiazolylbenzamide compound" or "compound (III)"); and
a compound including an N-benzylsuccinimide skeleton (hereinafter also referred to as an "N-benzylsuccinimide compound" or "compound (IV)").

### 4-Amino-1-phenylpyrazolopyrimidine compound represented by general formula (I)

The compound having an activity of promoting the intermolecular interaction and having an activity of increasing cholesterol in primary cilia of a cell in the present prophylactic or therapeutic agent for polycystic kidney disease is a 4-amino-1-phenylpyrazolopyrimidine compound represented by the following general formula (I): 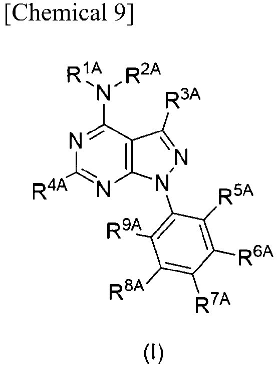
wherein R^{1A} and R^{2A} are the same or different and each represent:
   a hydrogen atom;
   a C1-6 alkyl group optionally having a substituent;
   a C1-6 alkoxy group optionally having a substituent;
   a C2-6 alkenyl group optionally having a substituent;
   a C2-6 alkynyl group optionally having a substituent;
   a phenyl group optionally having a substituent;
   an aliphatic cyclic group optionally having a substituent; or
   a heterocyclic group optionally having a substituent,
R^{3A} and R^{4A} are the same or different and each represent:
   a hydrogen atom;
   a halogen atom;
   a nitro group;
   a C1-6 alkyl group optionally having a substituent;
   a C1-6 alkoxy group optionally having a substituent; or
   a phenyl group optionally having a substituent, and
R^{5A}, R^{6A}, R^{7A}, R^{8A}, and R^{9A} are the same or different and each represent:
   a hydrogen atom;
   a halogen atom;
   a nitro group;
   a C1-6 alkyl group optionally having a substituent; or
   a C1-6 alkoxy group optionally having a substituent.

Each group of the 4-amino-1-phenylpyrazolopyrimidine compound represented by the general formula (I) in the present specification is described below.

The C1-6 alkyl group is not particularly limited as long as it is a linear or branched C1-6 alkyl group, and examples thereof include linear or branched alkyl groups having 1 to 6 carbon atoms, such as a methyl group, an ethyl group, an n-propyl group, an isopropyl group, an n-butyl group, an isobutyl group, an s-butyl group, a t-butyl group, an n-pentyl group, an isopentyl group, a neopentyl group, an n-hexyl group, and an isohexyl group. In the present specification, "n-" denotes normal, "s-" denotes secondary, and "t-" denotes tertiary.

The C1-6 alkoxy group is a group represented by RO- (wherein R is an alkyl group), and this "alkoxy group" is not particularly limited as long as it is a linear or branched C1-6 alkoxy group. Examples thereof include linear or branched alkoxy groups having 1 to 6 carbon atoms, such as a methoxy group, an ethoxy group, an n-propoxy group, an isopropoxy group, an n-butoxy group, an isobutoxy group, an s-butoxy group, a t-butoxy group, an n-pentyloxy group, an isopentyloxy group, a neopentyloxy group, an n-hexyloxy group, and an isohexyloxy group. The C1-6 alkoxy group is preferably a C1-4 alkoxy group, and more preferably a methoxy group or a t-butoxy group.

The C2-6 alkenyl group is not particularly limited, and examples thereof include linear or branched alkenyl groups having 2 to 6 carbon atoms, such as a vinyl group, a 1-propenyl group, an allyl group, an isopropenyl group, a 1-butenyl group, a 2-butenyl group, a 3-butenyl group, a 1-methyl-2-propenyl group, and a 1,3-butadienyl group.

Examples of the C2-6 alkynyl group includes linear or branched alkynyl groups having 2 to 6 carbon atoms, such as an ethynyl group, a 1-propynyl group, a 2-propynyl group, a 1-methyl-2-propynyl group, a 1-butynyl group, a 2-butynyl group, and a 3-butynyl group.

In the present specification, "substituent" in a C1-6 alkyl group optionally having a substituent, a C1-6 alkoxy group optionally having a substituent, a C2-6 alkynyl group optionally having a substituent, a C2-6 alkynyloxy group optionally having a substituent, a phenyl group optionally having a substituent, an aliphatic cyclic group optionally having a substituent, a heterocyclic group optionally having a substituent, a C3-8 cycloalkyl group optionally having a substituent, and a C3-8 cycloalkoxy group optionally having a substituent is not particularly limited, and examples thereof include:
a halogen atom;
a C1-6 alkyl group;
a C3-8 cycloalkyl group;
a C3-8 cycloalkenyl group;
a halo-C1-6 alkyl group;
a C1-6 alkoxy group;
a phenyl group optionally having a substituent;
a nitro group;
a 1-acetylazetidin-3-ylmethyl group;
a C3-8 cycloalkylcarbonylamino group; and
a C1-6 alkoxycarbonylamino group.
These groups may further include a substituent at any substitutable position, and examples of such a substituent include those similar to the substituents described above. Examples of the C1-6 alkyl group optionally having a substituent include a halo-C1-6 alkyl group. Examples of the halo-C1-6 alkyl group include a chloromethyl group, a chloroethyl group, a trifluoromethyl group, a 1,2-dichloro-n-propyl group, a 1-fluoro-n-butyl group, and a perfluoro-n-pentyl group. The C1-6 alkyl group optionally having a substituent described herein is not limited to these examples.

The C3-8 cycloalkyl group is not particularly limited, and examples thereof include a cyclopropyl group, a cyclobutyl group, a cyclopentyl group, a cyclohexyl group, a cycloheptyl group, and a cyclooctyl group.

Examples of the C3-8 cycloalkenyl group include a cyclopropenyl group, a cyclopentenyl group, a cyclohexenyl group, and a cyclooctenyl group. The double bond may be present at any position.

The halogen atom is not particularly limited, and examples thereof include a fluorine atom, a chlorine atom, a bromine atom, and an iodine atom.

In the 4-amino-1-phenylpyrazolopyrimidine compound represented by the general formula (I),
R^{1A} is preferably,
a C1-6 alkyl group optionally having a substituent, or
a phenyl group optionally having a substituent,
more preferably,
a C1-6 alkyl group having a C1-6 alkoxy group,
a C1-6 alkyl group having a phenyl group optionally having a substituent,
a C1-6 alkyl group having a C3-6 cycloalkenyl group, or
a phenyl group optionally having a substituent,
further preferably,
a C1-6 alkyl group having a methoxy group or a hydroxy group,
a C1-6 alkyl group having a phenyl group with a halogen atom at the 4th position,
a C1-6 alkyl group having a cyclohexenyl group, or
a phenyl group having one to three C1-6 alkyl groups, and
particularly preferably,
a 4-methoxypropyl group,
a 4-fluorobenzyl group,
a 3,5-dimethylphenyl group,
a 4-chlorophenylethyl group,
a (1-cyclohexenyl)ethyl group, or
a 4-hydroxy group.

In the 4-amino-1-phenylpyrazolopyrimidine compound represented by the general formula (I), R^{2A} is preferably a hydrogen atom.

In the 4-amino-1-phenylpyrazolopyrimidine compound represented by the general formula (I), R^{3A} is preferably a hydrogen atom.

In the 4-amino-1-phenylpyrazolopyrimidine compound represented by the general formula (I), R^{4A} is preferably a hydrogen atom.

In the 4-amino-1-phenylpyrazolopyrimidine compound represented by the general formula (I), R^{5A}, R^{6A}, R^{7A}, R^{8A}, and R^{9A} are each preferably a hydrogen atom or a C1-6 alkyl group, more preferably, a hydrogen atom or a methyl group.

Among these, preferable combinations include:
R^{5A} is a hydrogen atom, R^{6A} is a C1-6 alkyl group, R^{7A} is a hydrogen atom, R^{8A} is a hydrogen atom, and R^{9A} is a hydrogen atom;
R^{5A} is a C1-6 alkyl group, R^{6A} is a hydrogen atom, R^{7A} is a C1-6 alkyl group, R^{8A} is a hydrogen atom, and R^{9A} is a hydrogen atom; and
R^{5A} is a hydrogen atom, R^{6A} is a hydrogen atom, R^{7A} is a C1-6 alkoxy group, R^{8A} is a hydrogen atom, and R^{9A} is a hydrogen atom.

Specifically, preferable examples of the 4-amino-1-phenylpyrazolopyrimidine compound represented by the general formula (I) include:
a compound represented by the following formula (I-1) (4-methoxypropylamino-1-(2,4-dimethylphenyl)-1H-pyrazolo[3,4-d]pyrimidine);
a compound represented by the following formula (I-2) (4-(4-fluorobenzylamino)-1-(3-methylphenyl)-1H-pyrazolo[3,4-d]pyrimidine);
a compound represented by the following formula (I-3) (4-(3,5-dimethylphenyl)amino-1-(4-methoxyphenyl)-1H-pyrazolo[3,4-d]pyrimidine);
a compound represented by the following formula (I-4) (2-(4-chlorophenyl)ethylamino-1-(3-methylphenyl)-1H-pyrazolo[3,4-d]pyrimidine);
a compound represented by the following formula (I-5) (2-(1-cyclohexenyl)ethylamino-1-(3-methylphenyl)-1H-pyrazolo[3,4-d]pyrimidine); and
a compound represented by the following formula (I-6) (4-hydroxypropylamino-1-(2,4-dimethylphenyl)-1H-pyrazolo[3,4-d]pyrimidine).

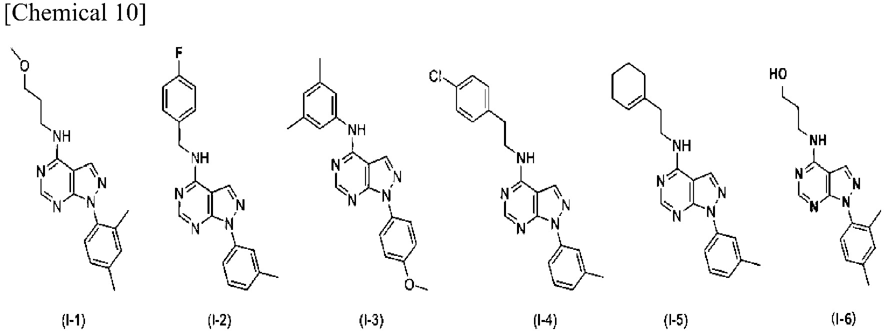

One or more of these 4-amino-1-phenylpyrazolopyrimidine compounds, or salts thereof, can be produced by known production methods.

The 4-amino-1-phenylpyrazolopyrimidine compounds can be produced, for example, using known compounds or commercially available products as starting materials. A specific production method is shown in a scheme 1 described below.

### 5-(Sulfamoyl)benzamide compound represented by general formula (II)

The compound having an activity of promoting the intermolecular interaction and having an activity of increasing cholesterol in primary cilia of a cell in the present prophylactic or therapeutic agent for polycystic kidney disease is a compound represented by the following general formula (II): 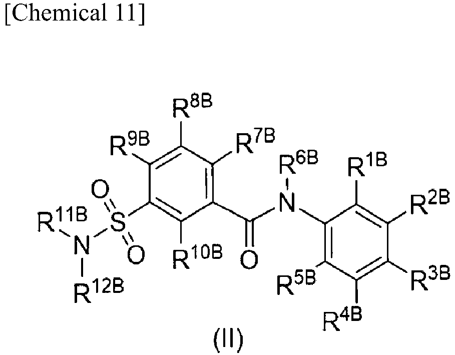
wherein R^{1B}, R^{2B}, R^{3B}, R^{4B}, R^{5B}, R^{7B}, R^{8B}, R^{9B}, and R^{10B} are the same or different and each represent:
   a hydrogen atom;
   a halogen atom;
   a nitro group;
   a C1-6 alkyl group optionally having a substituent;
   a C1-6 alkoxy group optionally having a substituent; or
   a phenyl group optionally having a substituent,
R^{6B} represents:
   a hydrogen atom; or
   a C1-6 alkyl group optionally having a substituent, and
R^{11B} and R^{12B} are the same or different and each represent:
   a hydrogen atom;
   a C1-6 alkyl group optionally having a substituent;
   a C1-6 alkoxy group optionally having a substituent;
   a C3-8 cycloalkyl group optionally having a substituent;
   a C3-6 cycloalkoxy group optionally having a substituent; or
   a phenyl group optionally having a substituent.

In the present specification, the C1-6 alkyl group, the C1-6 alkoxy group, the C3-8 cycloalkyl group, the halogen atom, and the substituent in the general formula (II) are the same as the C1-6 alkyl group, the C1-6 alkoxy group, the halogen atom, and the substituent in the general formula (I).

The C3-8 cycloalkoxy group is not particularly limited, and examples thereof include a cyclopropyloxy group, a cyclobutyloxy group, a cyclopentyloxy group, a cyclohexyloxy group, a cycloheptyloxy group, and a cyclooctyloxy group.

In the 5-(sulfamoyl)benzamide compound represented by the general formula (II), in the formula, at least one of R^{1B}, R^{2B}, R^{3B}, R4^{B}, and R^{5B} is a halogen atom, preferably a bromine atom, one to three of R^{1B}, R^{2B}, R^{3B}, R4^{B}, and R^{5B}, preferably two thereof, are methyl groups, and at least one of R^{2B} and R^{4B}, preferably both thereof, are methyl groups. Further, in the formula, R^{11B} is preferably a cyclohexyl group or a methoxyphenylmethyl group.

In the 5-(sulfamoyl)benzamide compound represented by the general formula (II), in the formula, R^{1B}, R^{2B}, R^{3B}, R^{4B}, and R^{5B} are each preferably a hydrogen atom, a halogen atom, or a C1-6 alkyl group, more preferably, a hydrogen atom, a bromine atom, or a methyl group.

Among these, particularly preferable combinations include:
R^{1B} is a hydrogen atom, R^{2B} is a hydrogen atom, R^{3B} is a halogen atom or a C1-6 alkyl group, R^{4B} is a hydrogen atom, and R^{5B} is a hydrogen atom; and
R^{1B} is a hydrogen atom, R^{2B} is a C1-6 alkyl group, R^{3B} is a hydrogen atom, R^{4B} is a C1-6 alkyl group, and R^{5B} is a hydrogen atom.

R^{7B}, R^{8B}, R^{9B}, and R^{10B} are each preferably a hydrogen atom, a halogen atom, or a C1-6 alkyl group, more preferably, a hydrogen atom, a chlorine atom, or a fluorine atom.

Among these, particularly preferable combinations include:
R^{7B} is a halogen atom, R^{8B} is a hydrogen atom, R^{9B} is a halogen atom or a C1-6 alkyl group, and R^{10B} is a hydrogen atom; and
R^{7B} is a chlorine atom or a fluorine atom, R^{8B} is a hydrogen atom, R^{9B} is a hydrogen atom, and R^{10B} is a hydrogen atom.

In the 5-(sulfamoyl)benzamide compound represented by the general formula (II), in the formula, R^{6B} is preferably a hydrogen atom.

In the 5-(sulfamoyl)benzamide compound represented by the general formula (II), in the formula, R^{11B} is preferably a hydrogen atom.

In the 5-(sulfamoyl)benzamide compound represented by the general formula (II), in the formula, R^{12B} is preferably a C1-6 alkyl group optionally having a substituent or a C1-6 cycloalkyl group optionally having a substituent, more preferably, a C1-6 alkyl group having a phenyl group bearing a C1-6 alkoxy group, or an unsubstituted C1-6 cycloalkyl group.

Preferable examples of the 5-(sulfamoyl)benzamide compound represented by the general formula (II) include the following compounds:
Compound (II-1): 2-chloro-N-(4-bromophenyl)-5-(cyclohexylaminosulfamoyl)benzamide
Compound (II-2): 2-fluoro-N-(3,5-dimethylphenyl)-5-[(1-methoxyphenylmethylamino)sulfamoyl]benzamide

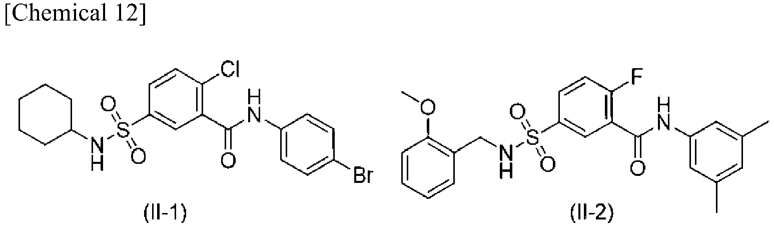

One or more of these 5-(sulfamoyl)benzamide compounds, or salts thereof, can be produced by known production methods.

The 5-(sulfamoyl)benzamide compounds can be produced, for example, using known compounds or commercially available products as starting materials. A specific production method is shown in a scheme 2 or 3 described below.

### N-Thiazolylbenzamide compound represented by general formula (III)

The compound having an activity of promoting the intermolecular interaction and having an activity of increasing cholesterol in primary cilia of a cell in the present prophylactic or therapeutic agent for polycystic kidney disease is an N-thiazolylbenzamide compound represented by the following general formula (III): 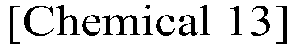 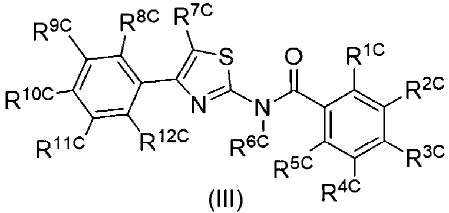
wherein R^{1C}, R^{2C}, R^{3C}, R^{4C}, R^{5C}, R^{8C}, R^{9C}, R^{10C}, R^{11C}, and R^{12C} are the same or different and each represent:
   a hydrogen atom;
   a halogen atom;
   a nitro group;
   a C1-6 alkyl group optionally having a substituent;
   a C1-6 alkoxy group optionally having a substituent; or
   a phenyl group optionally having a substituent,
R^{6C} represents:
   a hydrogen atom; or
   a C1-6 alkyl group optionally having a substituent, and
R^{7C} represents;
   a hydrogen atom;
   a halogen atom;
   a C1-6 alkyl group optionally having a substituent;
   a C1-6 alkoxy group optionally having a substituent; or
   a phenyl group optionally having a substituent.

In the present specification, the C1-6 alkyl group, the C1-6 alkoxy group, the halogen atom, and the substituent in the general formula (III) are the same as the C1-6 alkyl group, the C1-6 alkoxy group, the halogen atom, and the substituent in the general formula (I).

In the N-thiazolylbenzamide compound represented by the general formula (III), in the formula, at least one of R^{1C}, R^{2C}, R^{3C}, R^{4C}, and R^{5C} is preferably a nitro group, and R^{3C} is more preferably a nitro group. Further, in the formula, at least one of R^{8C}, R^{9C}, R^{10C}, R^{11C}, and R^{12C} is preferably a methyl group, and R^{10C} and/or R^{11C} are more preferably methyl groups. Further, in the formula, R^{1C}, R^{2C}, R^{4C}, R^{5C}, R^{7C}, R^{8C}, R^{9C}, and R^{12C} are preferably hydrogen atoms.

In the N-thiazolylbenzamide compound represented by the general formula (III), in the formula, R^{1C}, R^{2C}, R^{3C}, R^{4C}, and R^{5C} are each preferably a hydrogen atom, a halogen atom, a nitro group, or a C1-6 alkyl group, more preferably a hydrogen atom or a nitro group.

Among these, particularly preferable combinations include:
R^{1C} is a hydrogen atom, R^{2C} is a hydrogen atom, R^{3C} is a hydrogen atom, a halogen atom, a nitro group, or a C1-6 alkyl group, R^{4C} is a hydrogen atom, and R^{5C} is a hydrogen atom; and
R^{1C} is a hydrogen atom, R^{2C} is a C1-6 alkyl group, R^{3C} is a hydrogen atom, R^{4C} is a C1-6 alkyl group, and R^{5C} is a hydrogen atom.

In the N-thiazolylbenzamide compound represented by the general formula (III), in the formula, R^{6C} is preferably a hydrogen atom or a C1-6 alkyl group, more preferably a hydrogen atom.

In the N-thiazolylbenzamide compound represented by the general formula (III), in the formula, R^{7C} is preferably a hydrogen atom.

In the N-thiazolylbenzamide compound represented by the general formula (III), in the formula, R^{8C}, R^{9C}, R^{10C}, R^{11C}, and R^{12C} are each preferably a hydrogen atom or a C1-6 alkyl group, more preferably a hydrogen atom or a methyl group.

Among these, further preferable combinations include:
R^{8C} is a hydrogen atom, R^{9C} is a hydrogen atom, R^{10C} is a hydrogen atom or a C1-6 alkyl group, R^{11C} is a hydrogen atom or a C1-6 alkyl group, and R^{12C} is a hydrogen atom; and
particularly preferable combinations include:
R^{8C} is a hydrogen atom, R^{9C} is a hydrogen atom, R^{10C} is a C1-6 alkyl group, R^{11C} is a C1-6 alkyl group, and R^{12C} is a hydrogen atom.

Preferable examples of the N-thiazolylbenzamide compound represented by the general formula (III) includes the following compound:
Compound (III-1): 4-nitro-N-[4-(2,3-dimethylphenyl)-(2-thiazolyl)]benzamide

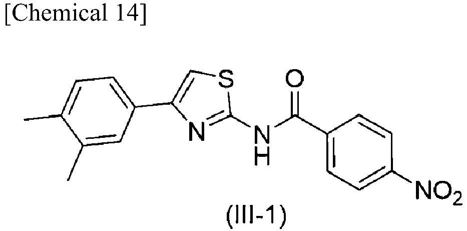

One or more of these N-thiazolylbenzamide compounds, or salts thereof, can be produced by known production methods.

The 5-(sulfamoyl)benzamide compounds can be produced, for example, using known compounds or commercially available products as starting materials. A specific production method is shown in a scheme 4 described below.

### N-Benzylsuccinimide compound represented by general formula (IV)

The compound having an activity of promoting the intermolecular interaction and having an activity of increasing cholesterol in primary cilia of a cell in the present prophylactic or therapeutic agent for polycystic kidney disease is an N-benzylsuccinimide compound represented by the following general formula (IV): 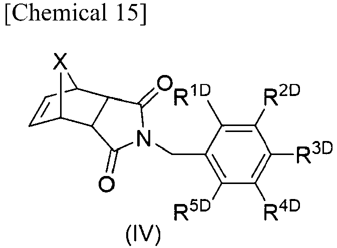 wherein R^{1D}, R^{2D}, R^{3D}, R^{4D}, and R^{5D} are the same or different and each represent a hydrogen atom or a C1-6 alkyl group optionally having a substituent, and X represents an oxygen atom or CH₂.

In the present specification, the C1-6 alkyl group, the C1-6 alkoxy group, the halogen atom, and the substituent in the general formula (IV) are the same as the C1-6 alkyl group, the C1-6 alkoxy group, the halogen atom, and the substituent in the general formula (I).

In the N-benzylsuccinimide compound represented by the general formula (IV), in the formula, R^{1D}, R^{2D}, R^{3D}, R^{4D}, and R^{5D} are each preferably a hydrogen atom, a halogen atom, a nitro group, or a C1-6 alkyl group, more preferably a hydrogen atom.

Among these, particularly preferable combinations include:
R^{1D} is a hydrogen atom, R^{2D} is a hydrogen atom, R^{3D} is a hydrogen atom, a halogen atom, a nitro group, or a C1-6 alkyl group, and R^{4D} is a hydrogen atom; and
R^{1D} is a hydrogen atom, R^{2D} is a hydrogen atom, R^{3D} is a hydrogen atom, R^{4D} is a hydrogen atom, and R^{5D} is a hydrogen atom.

In the N-benzylsuccinimide compound represented by the general formula (IV), in the formula, X is preferably an oxygen atom or CH₂, more preferably CH₂.

Preferable examples of the N-benzylsuccinimide compound represented by the general formula (IV) include the following compound (IV-1).

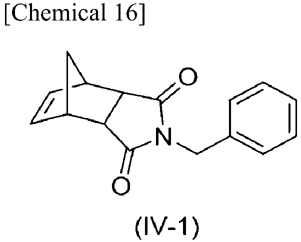

One or more of these N-benzylsuccinimide compounds, or salts thereof, can be produced by known production methods.

The N-benzylsuccinimide compounds can be produced, for example, using known compounds or commercially available products as starting materials. A specific production method is shown in a scheme 5 described below.

The salt in the present specification is not particularly limited as long as it is a pharmaceutically acceptable salt. Examples thereof include alkali metal salts (such as sodium salts and potassium salts), alkaline earth metal salts (such as calcium salts and magnesium salts), and salts with organic bases such as trimethylamine, triethylamine, pyridine, picoline, N-methylpyrrolidine, N-methylpiperidine, and N-methylmorpholine. When the compound is treated as a basic compound, examples of the salt include: acid addition salts with mineral acids such as hydrochloride, hydrobromide, hydroiodide, sulfate, nitrate, and phosphate; and acid addition salts with organic acids such as benzoate, methanesulfonate, ethanesulfonate, benzenesulfonate, p-toluenesulfonate, maleate, fumarate, tartrate, citrate, and acetate.

The compounds of the present invention and salts thereof may have one or more asymmetric centers in their structural formulae, and two or more optical isomers and diastereomers may exist. The present invention encompasses each of such optical isomers and mixtures thereof in any proportions. Further, the compounds of the present invention and salts thereof may have two geometric isomers derived from a carbon-carbon double bond in their structural formulae. The present invention also encompasses each of such geometric isomers and mixtures thereof in any proportions.

Further, the compounds of the present invention and salts thereof may exist in multiple tautomeric forms. The present invention encompasses each of such tautomers and mixtures thereof in any proportions.

Hereinafter, production methods for the compounds represented by the general formula (I) to general formula (IV) are exemplified.

### Production method of 4-amino-1-phenylpyrazolopyrimidine compound represented by general formula (I) (Production method (A))

The compound represented by the general formula (I) of the present invention can be produced, for example, by a production method shown in the following scheme 1. However, the present invention is not limited thereto. 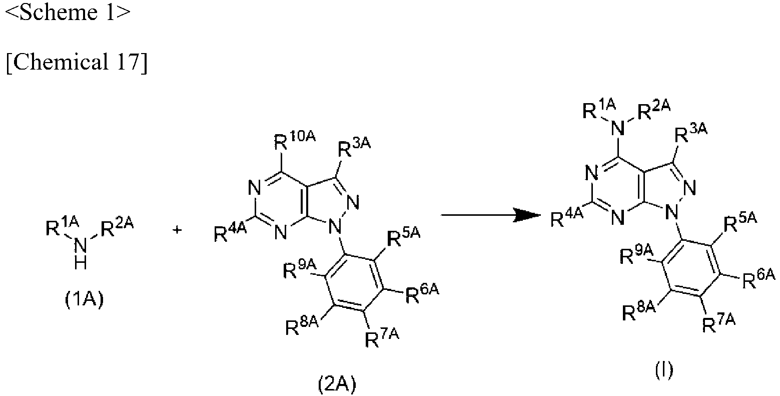 (wherein R^{1A}, R^{2A}, R^{3A}, R^{4A}, R^{5A}, R^{6A}, R^{7A}, R^{8A}, and R^{9A} are as defined above, and R^{10A} represents a halogen atom)

A production method (A-1) is a method for synthesizing a compound represented by the general formula (I), including a step of reacting a compound represented by a general formula (1A) with a compound represented by a general formula (2A) in a solvent in the presence of a base.

The amount of the compound represented by the general formula (1A) is not particularly limited as long as it is 1 equivalent or more relative to the compound represented by the general formula (2A), and the desired reaction proceeds. However, it is typically 1 equivalent or more and 200 equivalents or less.

Examples of the base used in the present reaction include: metal hydrides such as sodium hydride; organolithium compounds such as methyllithium, butyllithium, sec-butyllithium, t-butyllithium, and hexyllithium; and metal amides such as lithium diisopropylamide, lithium hexamethyldisilazane, sodium hexamethyldisilazane, and potassium hexamethyldisilazane.

The amount of the base used in the present reaction is not particularly limited as long as it is 1 equivalent or more relative to the compound represented by the general formula (1A), and the desired reaction proceeds. However, it is preferably 1 equivalent or more and 10 equivalents or less.

The solvent used in the present reaction is not particularly limited as long as the desired reaction proceeds. Examples thereof include: aromatic solvents such as benzene, toluene, xylene, mesitylene, chlorobenzene, and dichlorobenzene; and halogenated solvents such as dichloromethane, dichloroethane, chloroform, and carbon tetrachloride. These solvents may be used alone or in combination of two or more in any proportions.

The amount of the solvent used in the present reaction is not particularly limited as long as the desired reaction proceeds. However, it is typically 3 times by weight or more and 200 times by weight or less relative to the compound represented by the general formula (1A).

The temperature for carrying out the present reaction is not particularly limited as long as the desired reaction proceeds. However, it is typically 0°C or higher and 150°C or lower, or not higher than the boiling point of the solvent.

The time for carrying out the present reaction is not particularly limited as long as the desired reaction proceeds. However, it is typically 10 minutes or more and 24 hours or less.

As a post-treatment of the reaction, undissolved metals can be removed by filtration. Further, liquid-liquid separation can be carried out by adding water or an appropriate aqueous solution to the reaction mixture. When an aqueous solution is used, an acidic aqueous solution in which hydrochloric acid, sulfuric acid, ammonium chloride, or the like is dissolved; an alkaline aqueous solution in which potassium hydroxide, sodium hydroxide, sodium carbonate, potassium carbonate, sodium hydrogen carbonate, potassium hydrogen carbonate, or the like is dissolved; a saline solution; or the like may be used as appropriate. During the liquid-liquid separation, if necessary, a solvent immiscible with water may be added, such as aromatic solvents such as toluene, xylene, benzene, chlorobenzene, and dichlorobenzene; ester solvents such as ethyl acetate, isopropyl acetate, and butyl acetate; ether solvents such as diethyl ether, diisopropyl ether, and methyl t-butyl ether; halogenated solvents such as dichloromethane, dichloroethane, chloroform, and carbon tetrachloride; and hydrocarbon solvents such as hexane, heptane, cyclohexane, and methylcyclohexane. These solvents may be used alone or in combination of two or more in any proportions. The number of separations is not particularly limited and may be selected depending on the desired purity or yield. The liquid-liquid separation is not essential in the present reaction.

The reaction mixture including the compound represented by the general formula (I) obtained above may be dehydrated using a drying agent such as sodium sulfate or magnesium sulfate. However, this is not essential.

The reaction mixture including the compound represented by the general formula (I) obtained above can be subjected to solvent removal under reduced pressure, as long as the compound does not decompose.

The reaction mixture including the compound represented by the general formula (I) obtained after solvent removal can be purified by washing, reprecipitation, recrystallization, column chromatography, or the like using an appropriate solvent.

### Production method of 5-(sulfamoyl)benzamide compound represented by general formula (II) (Production method (B))

Hereinafter, a production method for the compound represented by the general formula (II) is exemplified.

The compound represented by the general formula (II) of the present invention can be produced, for example, by a production method shown in the following scheme 2 (production method (B-1)) or a production method shown in the following scheme 3 (production method (B-2)). However, the present invention is not limited thereto.

### Production method (B-1)

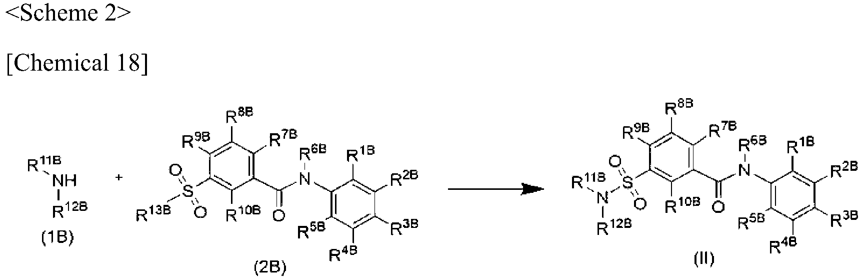

(wherein R^{1B}, R^{2B}, R^{3B}, R^{4B}, R^{5B}, R^{6B}, R^{7B}, R^{8B}, R^{9B}, R^{10B}, R^{11B}, and R^{12B} are as defined above, and R^{13B} represents a halogen atom)

A production Method (B-1) is a method for synthesizing a compound represented by the general formula (II), including a step of reacting an amine compound represented by a general formula (1B) with a compound represented by a general formula (2B) in a solvent in the presence of a base.

The amount of the compound represented by the general formula (1B) is not particularly limited as long as it is 1 equivalent or more relative to the compound represented by the general formula (2B), and the desired reaction proceeds. However, it is typically 1 equivalent or more and 200 equivalents or less.

Examples of the base used in the present reaction include: metal hydrides such as sodium hydride; organolithium compounds such as methyllithium, butyllithium, sec-butyllithium, t-butyllithium, and hexyllithium; and metal amides such as lithium diisopropylamide, lithium hexamethyldisilazane, sodium hexamethyldisilazane, and potassium hexamethyldisilazane.

The amount of the base used in the present reaction is not particularly limited as long as it is 1 equivalent or more relative to the compound represented by the general formula (1B), and the desired reaction proceeds. However, it is preferably 1 equivalent or more and 10 equivalents or less.

The solvent used in the present reaction is not particularly limited as long as the desired reaction proceeds. Examples thereof include: aromatic solvents such as benzene, toluene, xylene, mesitylene, chlorobenzene, and dichlorobenzene; and halogenated solvents such as dichloromethane, dichloroethane, chloroform, and carbon tetrachloride. These solvents may be used alone or in combination of two or more in any proportions.

The amount of the solvent used in the present reaction is not particularly limited as long as the desired reaction proceeds. However, it is typically 3 times by weight or more and 200 times by weight or less relative to the compound represented by the general formula (1B).

The temperature for carrying out the present reaction is not particularly limited as long as the desired reaction proceeds. However, it is typically 0°C or higher and 150°C or lower, or not higher than the boiling point of the solvent.

The time for carrying out the present reaction is not particularly limited as long as the desired reaction proceeds. However, it is typically 10 minutes or more and 24 hours or less.

As a post-treatment of the reaction, undissolved metals can be removed by filtration. Further, liquid-liquid separation can be carried out by adding water or an appropriate aqueous solution to the reaction mixture. When an aqueous solution is used, an acidic aqueous solution in which hydrochloric acid, sulfuric acid, ammonium chloride, or the like is dissolved; an alkaline aqueous solution in which potassium hydroxide, sodium hydroxide, sodium carbonate, potassium carbonate, sodium hydrogen carbonate, potassium hydrogen carbonate, or the like is dissolved; a saline solution; or the like may be used as appropriate. During the liquid-liquid separation, if necessary, a solvent immiscible with water may be added, such as aromatic solvents such as toluene, xylene, benzene, chlorobenzene, and dichlorobenzene; ester solvents such as ethyl acetate, isopropyl acetate, and butyl acetate; ether solvents such as diethyl ether, diisopropyl ether, and methyl t-butyl ether; halogenated solvents such as dichloromethane, dichloroethane, chloroform, and carbon tetrachloride; and hydrocarbon solvents such as hexane, heptane, cyclohexane, and methylcyclohexane. These solvents may be used alone or in combination of two or more in any proportions. The number of separations is not particularly limited and may be selected depending on the desired purity or yield. The liquid-liquid separation is not essential in the present reaction.

The reaction mixture including the compound represented by the general formula (II) obtained above may be dehydrated using a drying agent such as sodium sulfate or magnesium sulfate. However, this is not essential.

The reaction mixture including the compound represented by the general formula (II) obtained above can be subjected to solvent removal under reduced pressure, as long as the compound does not decompose.

The reaction mixture including the compound represented by the general formula (II) obtained after solvent removal can be purified by washing, reprecipitation, recrystallization, column chromatography, or the like using an appropriate solvent.

### Production method (B-2)

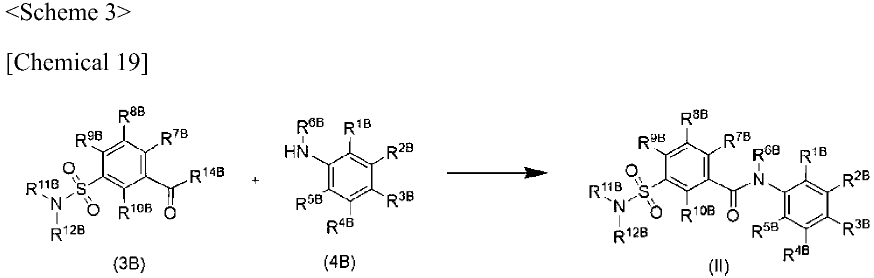

(wherein R^{1B}, R^{2B}, R^{3B}, R^{4B}, R^{5B}, R^{6B}, R^{7B}, R^{8B}, R^{9B}, R^{10B} R^{11B}, and R^{12B} are as defined above, and R^{14B} represents a halogen atom)

A production method (B-2) is a method for synthesizing a compound represented by the general formula (II), including a step of reacting a sulfonamide compound represented by a general formula (3B) with an amine compound represented by a general formula (4B) in a solvent in the presence of a base.

The amount of the compound represented by the general formula (4B) is not particularly limited as long as it is 1 equivalent or more relative to the compound represented by the general formula (3B), and the desired reaction proceeds. However, it is typically 1 equivalent or more and 200 equivalents or less.

Examples of the base used in the present reaction include: metal hydrides such as sodium hydride; organolithium compounds such as methyllithium, butyllithium, sec-butyllithium, t-butyllithium, and hexyllithium; and metal amides such as lithium diisopropylamide, lithium hexamethyldisilazane, sodium hexamethyldisilazane, and potassium hexamethyldisilazane.

The amount of the base used in the present reaction is not particularly limited as long as it is 1 equivalent or more relative to the compound represented by the general formula (4B), and the desired reaction proceeds. However, it is preferably 1 equivalent or more and 10 equivalents or less.

The solvent used in the present reaction is not particularly limited as long as the desired reaction proceeds. Examples thereof include: aromatic solvents such as benzene, toluene, xylene, mesitylene, chlorobenzene, and dichlorobenzene; and halogenated solvents such as dichloromethane, dichloroethane, chloroform, and carbon tetrachloride. These solvents may be used alone or in combination of two or more in any proportions.

The amount of the solvent used in the present reaction is not particularly limited as long as the desired reaction proceeds. However, it is typically 3 times by weight or more and 200 times by weight or less relative to the compound represented by the general formula (3B).

The temperature for carrying out the present reaction is not particularly limited as long as the desired reaction proceeds. However, it is typically 0°C or higher and 150°C or lower, or not higher than the boiling point of the solvent.

The time for carrying out the present reaction is not particularly limited as long as the desired reaction proceeds. However, it is typically 10 minutes or more and 24 hours or less.

As a post-treatment of the reaction, undissolved metals can be removed by filtration. Further, liquid-liquid separation can be carried out by adding water or an appropriate aqueous solution to the reaction mixture. When an aqueous solution is used, an acidic aqueous solution in which hydrochloric acid, sulfuric acid, ammonium chloride, or the like is dissolved; an alkaline aqueous solution in which potassium hydroxide, sodium hydroxide, sodium carbonate, potassium carbonate, sodium hydrogen carbonate, potassium hydrogen carbonate, or the like is dissolved; a saline solution; or the like may be used as appropriate. During the liquid-liquid separation, if necessary, a solvent immiscible with water may be added, such as aromatic solvents such as toluene, xylene, benzene, chlorobenzene, and dichlorobenzene; ester solvents such as ethyl acetate, isopropyl acetate, and butyl acetate; ether solvents such as diethyl ether, diisopropyl ether, and methyl t-butyl ether; halogenated solvents such as dichloromethane, dichloroethane, chloroform, and carbon tetrachloride; and hydrocarbon solvents such as hexane, heptane, cyclohexane, and methylcyclohexane. These solvents may be used alone or in combination of two or more in any proportions. The number of separations is not particularly limited and may be selected depending on the desired purity or yield. The liquid-liquid separation is not essential in the present reaction.

The reaction mixture including the compound represented by the general formula (II) obtained above may be dehydrated using a drying agent such as sodium sulfate or magnesium sulfate. However, this is not essential.

The reaction mixture including the compound represented by the general formula (II) obtained above can be subjected to solvent removal under reduced pressure, as long as the compound does not decompose.

The reaction mixture including the compound represented by the general formula (II) obtained after solvent removal can be purified by washing, reprecipitation, recrystallization, column chromatography, or the like using an appropriate solvent.

### Production method of N-thiazolylbenzamide compound represented by general formula (III) (Production method (C))

Hereinafter, a production method for the compound represented by the general formula (III) is exemplified.

The compound represented by the general formula (III) of the present invention can be produced, for example, by a production method shown in the following scheme 4. However, the present invention is not limited thereto. 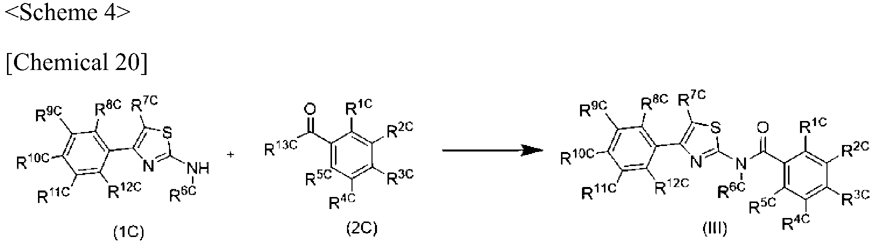 (wherein R^{1C}, R^{2C}, R^{3C}, R^{4C}, R^{5C}, R^{6C}, R^{7C}, R^{8C}, R^{9C}, R^{10C}, R^{11C}, and R^{12C} are as defined above, and R^{13C} represents a halogen atom)

A production method (C) is a method for synthesizing a compound represented by the general formula (III), including a step of reacting a thiazole compound represented by a general formula (1C) with a compound represented by a general formula (2C) in a solvent in the presence of a base.

The amount of the compound represented by the general formula (1C) is not particularly limited as long as it is 1 equivalent or more relative to the compound represented by the general formula (2C), and the desired reaction proceeds. However, it is typically 1 equivalent or more and 200 equivalents or less.

Examples of the base used in the present reaction include metal hydrides such as sodium hydride; organolithium compounds such as methyllithium, butyllithium, sec-butyllithium, t-butyllithium, and hexyllithium; and metal amides such as lithium diisopropylamide, lithium hexamethyldisilazane, sodium hexamethyldisilazane, and potassium hexamethyldisilazane.

The amount of the base used in the present reaction is not particularly limited as long as it is 1 equivalent or more relative to the compound represented by the general formula (1C), and the desired reaction proceeds. However, it is preferably 1 equivalent or more and 10 equivalents or less.

The solvent used in the present reaction is not particularly limited as long as the desired reaction proceeds. Examples thereof include aromatic solvents such as benzene, toluene, xylene, mesitylene, chlorobenzene, and dichlorobenzene; and halogenated solvents such as dichloromethane, dichloroethane, chloroform, and carbon tetrachloride. These solvents may be used alone or in combination of two or more in any proportions.

The amount of the solvent used in the present reaction is not particularly limited as long as the desired reaction proceeds. However, it is typically 3 times by weight or more and 200 times by weight or less relative to the compound represented by the general formula (1C).

The temperature for carrying out the present reaction is not particularly limited as long as the desired reaction proceeds. However, it is typically 0°C or higher and 150°C or lower, or not higher than the boiling point of the solvent.

The time for carrying out the present reaction is not particularly limited as long as the desired reaction proceeds. However, it is typically 10 minutes or more and 24 hours or less.

As a post-treatment of the reaction, undissolved metals can be removed by filtration. Further, liquid-liquid separation can be carried out by adding water or an appropriate aqueous solution to the reaction mixture. When an aqueous solution is used, an acidic aqueous solution in which hydrochloric acid, sulfuric acid, ammonium chloride, or the like is dissolved; an alkaline aqueous solution in which potassium hydroxide, sodium hydroxide, sodium carbonate, potassium carbonate, sodium hydrogen carbonate, potassium hydrogen carbonate, or the like is dissolved; a saline solution; or the like may be used as appropriate. During the liquid-liquid separation, if necessary, a solvent immiscible with water may be added, such as aromatic solvents such as toluene, xylene, benzene, chlorobenzene, and dichlorobenzene; ester solvents such as ethyl acetate, isopropyl acetate, and butyl acetate; ether solvents such as diethyl ether, diisopropyl ether, and methyl t-butyl ether; halogenated solvents such as dichloromethane, dichloroethane, chloroform, and carbon tetrachloride; and hydrocarbon solvents such as hexane, heptane, cyclohexane, and methylcyclohexane. These solvents may be used alone or in combination of two or more in any proportions. The number of separations is not particularly limited and may be selected depending on the desired purity or yield. The liquid-liquid separation is not essential in the present reaction.

The reaction mixture including the compound represented by the general formula (III) obtained above may be dehydrated using a drying agent such as sodium sulfate or magnesium sulfate. However, this is not essential.

The reaction mixture including the compound represented by the general formula (III) obtained above can be subjected to solvent removal under reduced pressure, as long as the compound does not decompose.

The reaction mixture including the compound represented by the general formula (III) obtained after solvent removal can be purified by washing, reprecipitation, recrystallization, column chromatography, or the like using an appropriate solvent.

### Production method of N-benzylsuccinimide compound represented by general formula (IV) (Production method (D))

Hereinafter, a production method for the compound represented by the general formula (IV) is exemplified.

The compound represented by the general formula (IV) of the present invention can be produced, for example, by a production method shown in the following scheme 5. However, the present invention is not limited thereto. 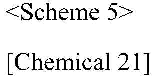 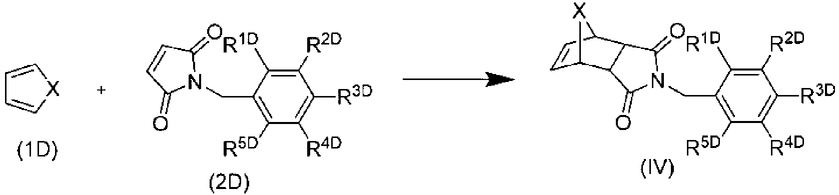 (wherein R^{1D}, R^{2D}, R^{3D}, R^{4D}, and R^{5D} are as defined above, and X represents an oxygen atom or CH₂)

A production method (D) is a method for synthesizing an N-benzylsuccinimide compound represented by the general formula (IV), including a step of reacting a compound represented by a general formula (1D) with an N-maleimide compound represented by a general formula (2D) without a solvent or in a solvent.

The amount of the compound represented by the general formula (1D) is not particularly limited as long as it is 1 equivalent or more relative to the compound represented by the general formula (2D), and the desired reaction proceeds. However, it is typically 1 equivalent or more and 200 equivalents or less.

When a solvent is used in the present reaction, the type of the solvent is not particularly limited as long as the desired reaction proceeds. Examples thereof include aromatic solvents such as benzene, toluene, xylene, mesitylene, chlorobenzene, and dichlorobenzene; and halogenated solvents such as dichloromethane, dichloroethane, chloroform, and carbon tetrachloride. These solvents may be used alone or in combination of two or more in any proportions.

The amount of the solvent used in the present reaction is not particularly limited as long as the desired reaction proceeds. However, it is typically 3 times by weight or more and 200 times by weight or less relative to the compound represented by the general formula (1D).

In the present reaction, an additive such as a catalyst may be added as necessary.

The temperature for carrying out the present reaction is not particularly limited as long as the desired reaction proceeds. However, it is typically -78°C or higher and 250°C or lower, or not higher than the boiling point of the solvent.

The time for carrying out the present reaction is not particularly limited as long as the desired reaction proceeds. However, it is typically 10 minutes or more and 24 hours or less.

As a post-treatment of the reaction, undissolved metals can be removed by filtration. Further, liquid-liquid separation can be carried out by adding water or an appropriate aqueous solution to the reaction mixture. When an aqueous solution is used, an acidic aqueous solution in which hydrochloric acid, sulfuric acid, ammonium chloride, or the like is dissolved; an alkaline aqueous solution in which potassium hydroxide, sodium hydroxide, sodium carbonate, potassium carbonate, sodium hydrogen carbonate, potassium hydrogen carbonate, or the like is dissolved; a saline solution; or the like may be used as appropriate. During the liquid-liquid separation, if necessary, a solvent immiscible with water may be added, such as aromatic solvents such as toluene, xylene, benzene, chlorobenzene, and dichlorobenzene; ester solvents such as ethyl acetate, isopropyl acetate, and butyl acetate; ether solvents such as diethyl ether, diisopropyl ether, and methyl t-butyl ether; halogenated solvents such as dichloromethane, dichloroethane, chloroform, and carbon tetrachloride; and hydrocarbon solvents such as hexane, heptane, cyclohexane, and methylcyclohexane. These solvents may be used alone or in combination of two or more in any proportions. The number of separations is not particularly limited and may be selected depending on the desired purity or yield. The liquid-liquid separation is not essential in the present reaction.

The reaction mixture including the compound represented by the general formula (IV) obtained above may be dehydrated using a drying agent such as sodium sulfate or magnesium sulfate. However, this is not essential.

The reaction mixture including the compound represented by the general formula (IV) obtained above can be subjected to solvent removal under reduced pressure, as long as the compound does not decompose.

The reaction mixture including the compound represented by the general formula (IV) obtained after solvent removal can be purified by washing, reprecipitation, recrystallization, column chromatography, or the like using an appropriate solvent.

The subjects to which the present prophylactic or therapeutic agent for polycystic kidney disease is to be administered include mammals, specifically humans, cats, dogs, monkeys, cattle, horses, mice, rats, hamsters, guinea pigs, rabbits, goats, pigs, and sheep.

Examples of the subjects to which the present prophylactic or therapeutic agent for polycystic kidney disease is to be administered include subjects diagnosed with polycystic kidney disease or subjects diagnosed as requiring prevention of polycystic kidney disease. The agent may also be a prophylactic or therapeutic agent for autosomal dominant polycystic kidney disease for administration to a subject having a gene mutation in PKD1 and/or PKD2.

The present prophylactic or therapeutic agent for polycystic kidney disease may include a pharmaceutically acceptable additive. Examples of the additive include: physiological saline, buffered physiological saline, dextrose, water for injection, glycerol, ethanol, and a combination thereof; a stabilizer, a solubilizer, and a surfactant; and a buffer, a preservative, a tonicity agent, an excipient, and a lubricant.

The dose of the present prophylactic or therapeutic agent for polycystic kidney disease to be administered to a subject varies depending on age, body weight, symptoms, therapeutic effects, route of administration, treatment period, and the like. However, for a typical adult, the dose per administration is in a range of 0.1 mg to 1000 mg, preferably 1 mg to 500 mg, in terms of the amount of the compound as an active ingredient. The administration may be carried out four times daily, three times daily, twice daily, or once daily; or at intervals of every other day, every two days, every three days, every four days, every five days, every seven days, every eight days, or every nine days; or twice a week, once a month, or twice a month, each independently. The route of administration is not particularly limited, and examples thereof include oral administration, intravenous administration, intramuscular administration, subcutaneous administration, transdermal administration, and intranasal administration.

The present prophylactic or therapeutic agent for polycystic kidney disease may be used in combination with other agents for kidney diseases, as well as antihypertensive agents, lipid-lowering agents, antidiabetic agents, and the like.

Hereinafter, the present invention is described in more detail by way of Examples. However, the technical scope of the present invention is not limited to these examples.

### [Example 1] Screening of tool compounds

In order to perform screening of tool compounds, the present inventors constructed a screening platform incorporating a split-luciferase system targeting the intermolecular interaction between ORP and VAP-A, and used the platform to search for compounds that promote the intermolecular interaction between ORP3 and VAPA. Specifically, polynucleotides encoding ORP3 or VAPA were first inserted into expression vectors included in the NanoBiT (registered trademark) Protein:Protein Interaction System (Promega Corp.), and the resulting vectors were introduced into HEK293T cells by lipofection. Drug selection with neomycin was then performed to obtain stable HEK293T cell clones expressing the split luciferase (HEK293T cells constitutively expressing the split luciferase for detecting the interaction between ORP3 and VAPA). Next, the above-obtained split-luciferase stably expressing HEK293T cells or parental HEK293 cells were seeded into 96-well plates. After 24 hours of culture, library compounds, and Nano-Glo Live Cell Reagent (Promega Corp.), which is a luminescent substrate for split luciferase, were added to culture. Immediately after treatment, the plates were set in a luminescent image analyzer (LAS-4000, GE HealthCare), and images were continuously acquired for up to 4 hours after substrate addition. The acquired images were analyzed using Multi Gauge software attached to the LAS-4000 to obtain quantitative luminescence data for each well. In the above split-luciferase expression system, a Large BiT (LgBiT; 17.6 kDa) was fused to ORP3, and a Small BiT (SmBiT; 11 amino acids) was fused to VAP-A, such that luciferase is reconstituted upon interaction between ORP3 and VAP-A. The split-luciferase expression system was thus constructed.

At both ends of the 96-well plate, DMSO used as a solvent for diluting the compounds was placed as a negative control, and as a positive control, 10 µM phorbol 12-myristate 13-acetate (PMA), which showed enhancement of luminescence in the ORP3-VAPA split-luciferase system, was used. It was confirmed that, upon treatment with PMA, the enhancement of luminescence was saturated within 2 hours after treatment, and the luminescence was sustained for up to 4 hours after treatment. Further, the enhancement effect of luminescence at 2 hours after treatment was approximately 5-fold.

Next, 10,880 compounds from the ChemDiv diversity library (ChemDiv), part of the compound library held by Kyoto University (a National University Corporation) were screened at a concentration of 10 µM. Compounds showing a luminescence enhancement effect with a relative level of 2.8 or higher, taking the luminescence level in the absence of compound treatment as 1, were selected as hit compounds, resulting in a total of 44 hit compounds. Each of the hit compounds was designated as compounds A to Z and AA to AR.

The results are shown in FIG. 1. The maximum enhancement effect was 4.4-fold. In addition, when the concentration dependency of the luminescence enhancement effect was examined for the hit compounds, all compounds showed an enhancement effect at 3 µM.

### [Example 2] Increase in cholesterol in primary cilia

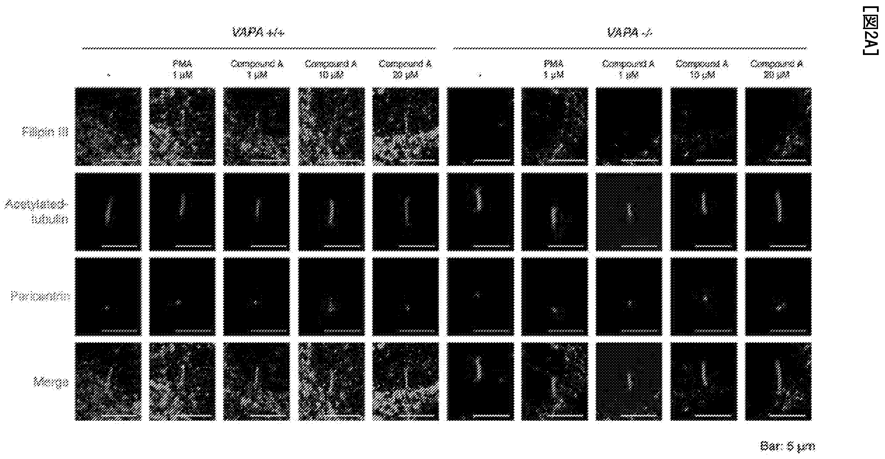

Among the hit compounds obtained in Example 1, 10 compounds (compounds A, C, D, I, R, V, AB, AF, AL, and AP) were evaluated for their ability to increase cholesterol in primary cilia. hTERT-immortalized human retinal pigment epithelial cells (hTERT-RPE1 cells: ATCC CRL-4000) were treated with the hit compounds at 37°C for 4 hours, followed by multiplex staining with the cholesterol probe Filipin III (Merck KGaA), anti-acetylated tubulin antibodies (Merck KGaA) as a primary cilium marker, and anti-pericentrin antibodies (Bethyl Laboratories, Inc.) as a centrosome marker. The results of observation of cholesterol in primary cilia using a confocal laser microscope and the results of measurement of Filipin III fluorescence intensity in primary cilia are shown in FIG. 2A to FIG. 4B. In addition, the same analysis was performed using VAP-A-deficient hTERT-RPE1 cells generated by deleting the VAP-A gene using the CRISPR-ObLiGaRe method established by Royba, Miyamoto et al. (Sci Rep 2017). In the drawings, VAPA+/+ indicates hTERT-RPE1 cells, and VAPA-/- indicates VAP-A-deficient hTERT-RPE1 cells.

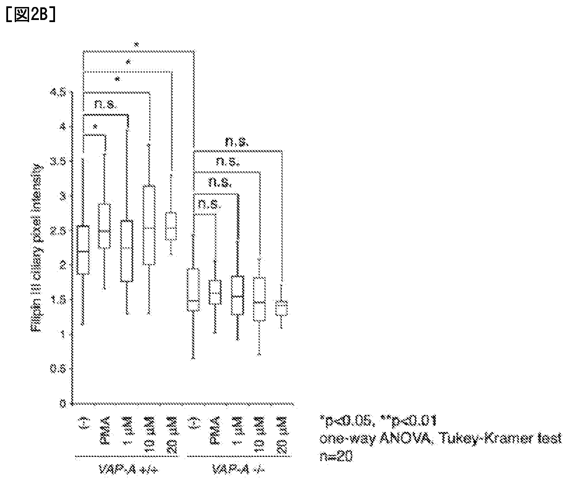

As shown in FIGS. 2A and 2B, it was revealed by Filipin III staining and immunostaining that cholesterol in the primary cilia of hTERT-RPE1 cells increased upon treatment with the compound A. The increase in cholesterol in the primary cilia induced by the compound A, in other words, the cholesterol delivery activity of the compound A to the primary cilia, was not detected in VAP-A-deficient cells (VAP-A-/-), indicating that the above activity is dependent on ORP3/VAPA. PMA is a PKC activator, and it has been reported that phosphorylation of ORP3 by PKC promotes its intermolecular interaction with VAP-A. Thus, in the present example, PMA was used as a positive control.

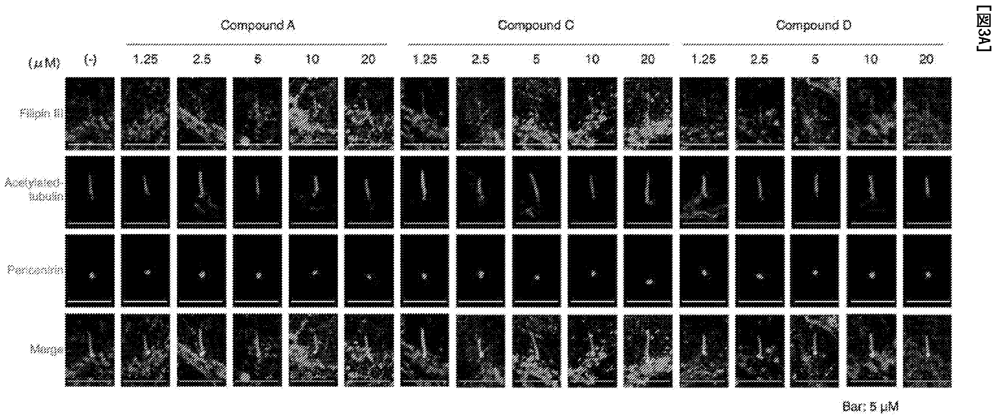

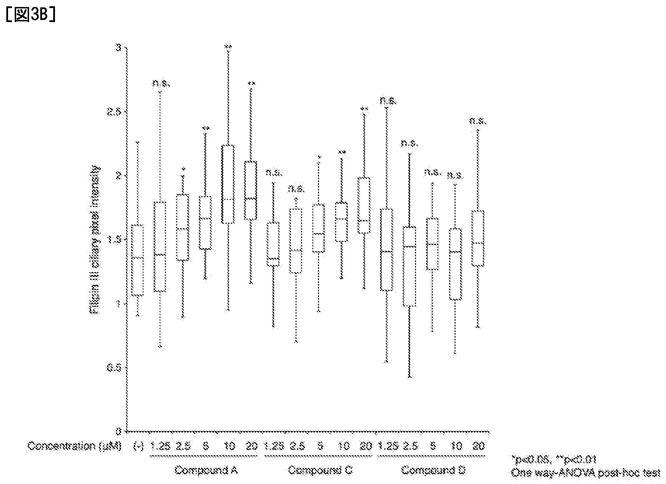

As shown in FIGS. 3A and 3B, it was revealed by Filipin III staining and immunostaining that cholesterol in the primary cilia of hTERT-RPE1 cells increased in a dose-dependent manner upon treatment with the compounds A and C. In contrast, the compound D did not show activity to increase cholesterol in the primary cilia.

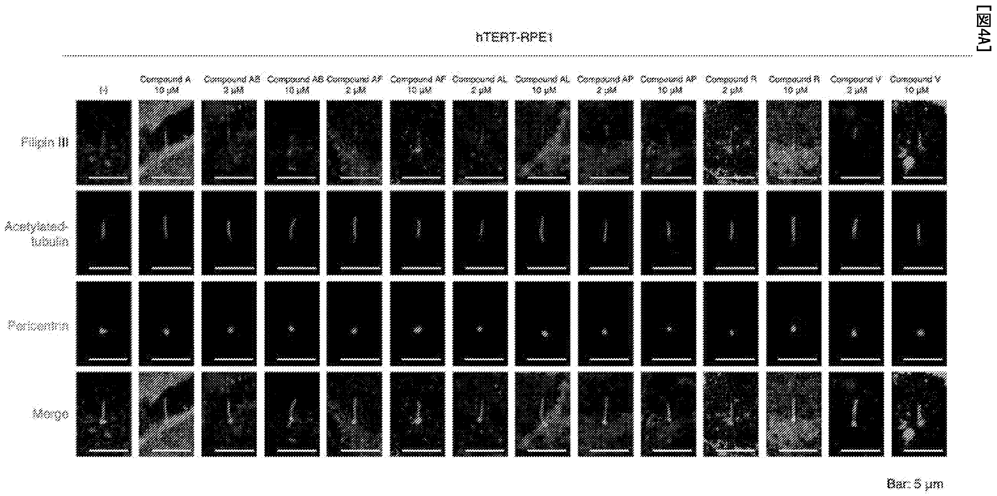

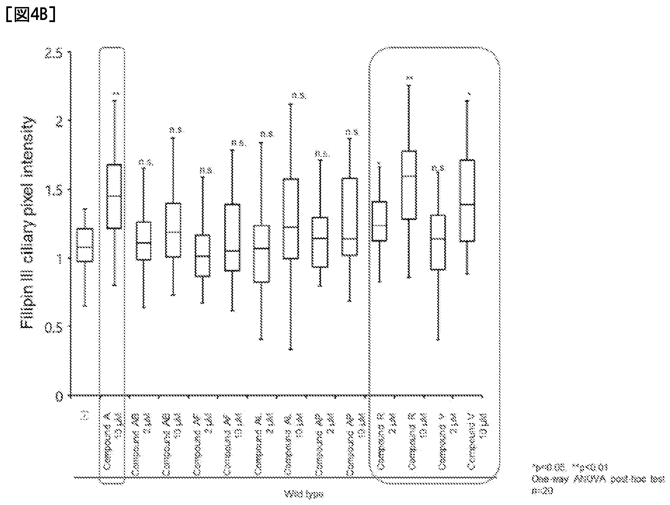

Further, as shown in FIGS. 4A and 4B, it was revealed by Filipin III staining and immunostaining that cholesterol in the primary cilia of hTERT-RPE1 cells increased in a dose-dependent manner upon treatment with the compounds A, R, and V. The compounds AB, AF, AL, and AP did not show activity to increase cholesterol in the primary cilia.

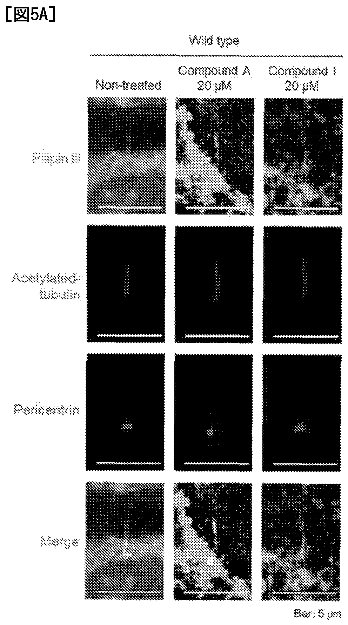

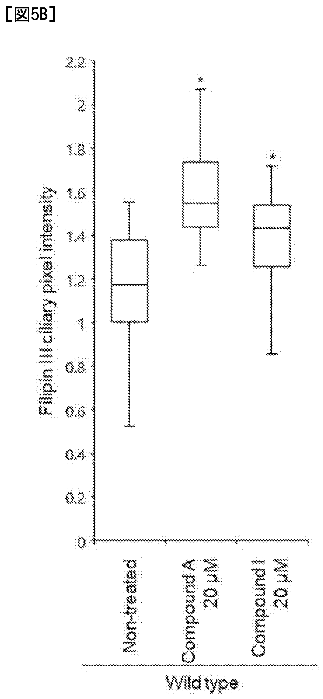

As shown in FIGS. 5A and 5B, it was revealed by Filipin III staining and immunostaining that cholesterol in the primary cilia of hTERT-RPE1 cells also increased upon treatment with the compound I.

From the above results, it was demonstrated that the compounds A, C, I, R, and V significantly increased cholesterol delivery to the primary cilia in a dose-dependent manner. Importantly, since these activities were not observed in VAP-A-deficient hTERT-RPE1 cells, it was genetically demonstrated that the increase in cholesterol in the primary cilia induced by the compounds A, C, I, R, and V is dependent on ORP3/VAP-A.

The compounds A, C, I, and R are shown below. The compound A corresponds to the above-mentioned compound (III-1), the compound C corresponds to the above-mentioned compound (IV-1), the compound I corresponds to the above-mentioned compound (II-2), and the compound R corresponds to the above-mentioned compound (I-5).

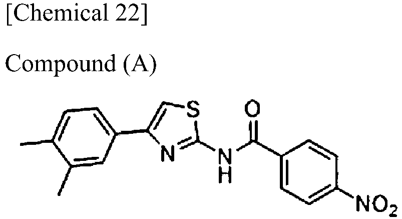

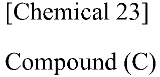

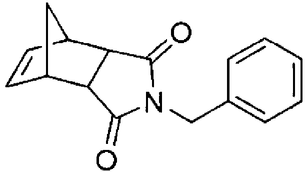

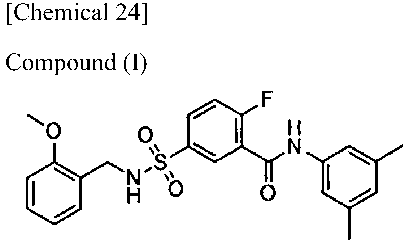

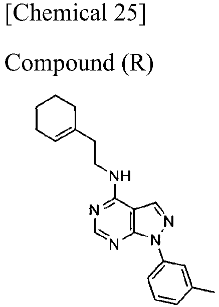

### [Example 3] Restoration of primary ciliary localization of polycystin 1 missense protein

Using mouse kidney collecting duct-derived cells (mIMCD3 cells: ATCC Cat. No. CRL-2123) expressing a polycystin 1 missense protein (a mutation in which cysteine at position 210 in the amino acid sequence of SEQ ID NO: 1 is substituted with glycine), it was examined whether the compound C has an activity to restore localization of polycystin 1 to primary cilia.

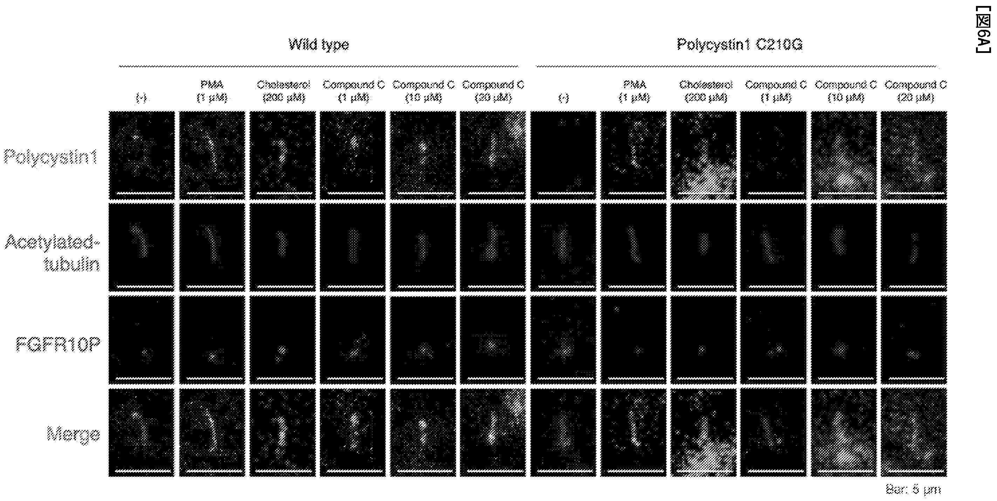

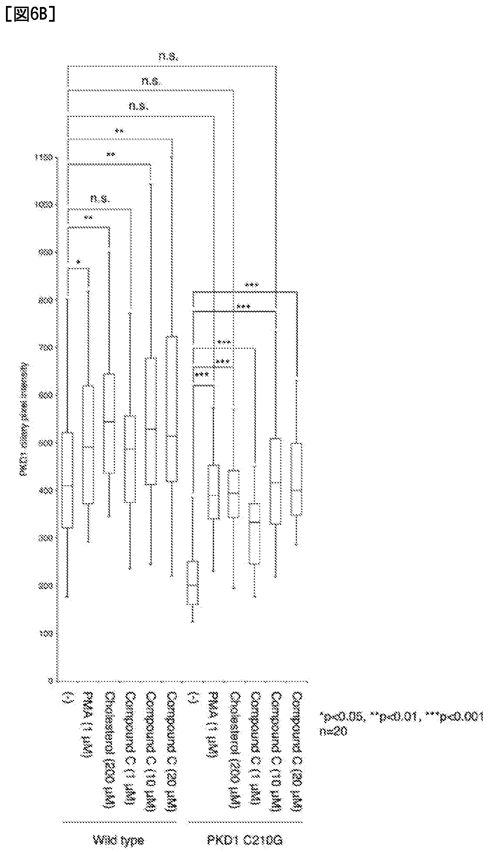

The above mutant mIMCD3 cells were treated with the compound C at 37°C for 4 hours, followed by multiplex staining with anti-polycystin 1 antibodies (Santa Cruz Biotechnology Inc.), anti-acetylated tubulin antibodies (Merck KGaA) as a primary cilium marker, and anti-FGFR10P antibodies (Proteintech Group, Inc.) as a centrosome marker. The results of observation of cholesterol in primary cilia using a confocal laser microscope and the results of measurement of fluorescence intensity of polycystin 1 protein in primary cilia are shown in FIGS. 6A and 6B. As a control, mIMCD3 cells without mutations were used (Wild type).

As shown in FIGS. 6A and 6B, it was confirmed that the compound C has an activity to restore localization of polycystin 1 to the primary cilia even when the polycystin 1 protein is mutated.

### [Example 4] Inhibitory effect on tubule diameter enlargement in cyst culture system reconstituting cyst formation via 3D culture of renal tubular cells: Compounds A, C, R, and V

Using mutant mIMCD3 cells expressing a polycystin 1 missense protein or mutant mIMCD3 cells expressing a polycystin 2 missense protein, it was examined whether each compound has an activity of suppressing cyst enlargement. The mutation in the polycystin 1 protein or polycystin 2 protein reduces primary ciliary activity and results in cyst enlargement.

Three-dimensional cultures of the mutant mIMCD3 cells were prepared in accordance with the method described by Rachel H. Giles et al. (Nature Protocols, volume 9, pages 2725-2731 (2014)). In mIMCD3 cells (mIMCD3 cells: ATCC Cat. No. CRL-2123), PC1 mutant mouse kidney collecting duct-derived cells (PC1 mutant mIMCD3 cells) expressing a polycystin 1 missense protein (a mutation in which cysteine at position 210 in the amino acid sequence of SEQ ID NO: 1 is substituted with glycine), or PC2 mutant mIMCD3 cells expressing a polycystin 2 missense protein (a mutation in which tryptophan at position 412 in the amino acid sequence of SEQ ID NO: 2 (corresponding to position 414 in a human polycystin 2 missense protein) is substituted with glycine) were prepared. Each of the mutant mIMCD3 cells was suspended in DMEM/F-12 (L-glutamine) medium supplemented with 10% (vol/vol) FBS and 1% (vol/vol) penicillin-streptomycin, mixed with the compound A, C, R, or V at 20 µM or cholesterol at 200 µM, and Matrigel, and then transferred to an 8-well chamber slide plate. The mixture was incubated at 37°C under 5% CO₂ conditions until solidification (15 to 30 minutes). Thereafter, pre-warmed medium was added to the solidified cell-Matrigel layer, and the cells were incubated at 37°C under 5% CO₂ conditions with medium replacement until cyst formation.

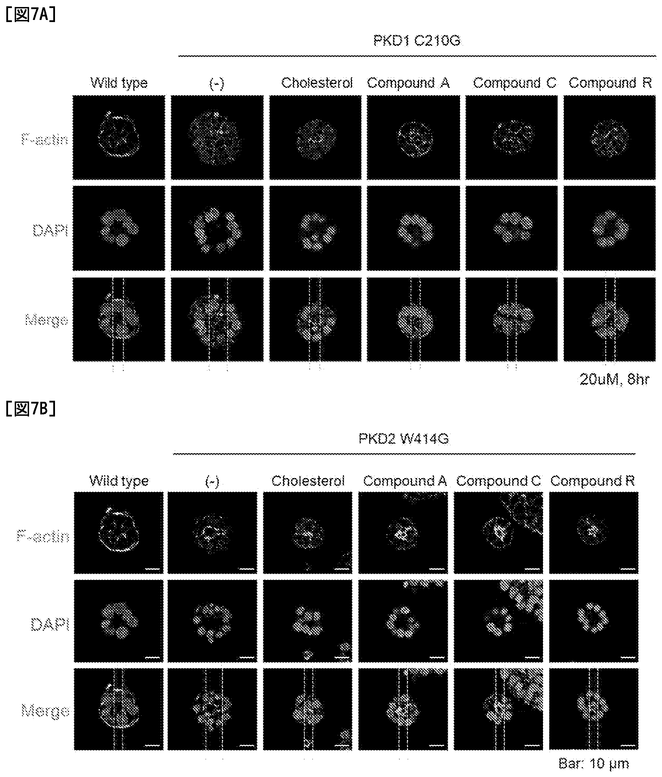

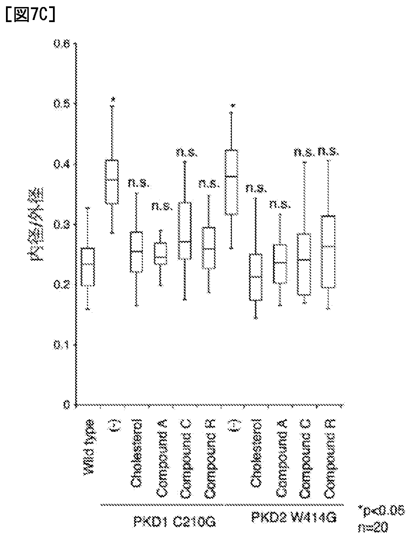

The cysts thus formed were fixed, and sections were prepared. Subsequently, F-actin was stained with phalloidin, and DNA was stained with DAPI, followed by imaging using a fluorescence microscope. Further, in the merged images of the formed cysts, the size of the epithelial lumen formed inside the cyst was defined as the inner diameter, and the overall size of the cyst was defined as the outer diameter, and an inner diameter/outer diameter ratio was calculated. The results are shown in FIGS. 7A to 7C. In FIG. 7A, the dotted line in the merged image indicates the width of the inner diameter.

As shown in FIGS. 7A to 7C, the compounds A, C, and R reduced the inner diameter/outer diameter ratio to a level comparable to a level similar to that observed with cholesterol treatment, confirming that the compounds have an activity of suppressing cyst size.

### [Example 5] Inhibitory effect on tubule diameter enlargement in cyst culture system reconstituting cyst formation via 3D culture of renal tubular cells: Comparison with tolvaptan

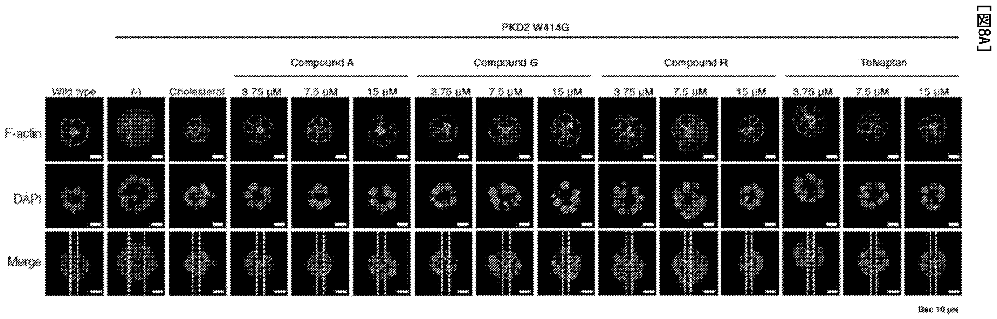

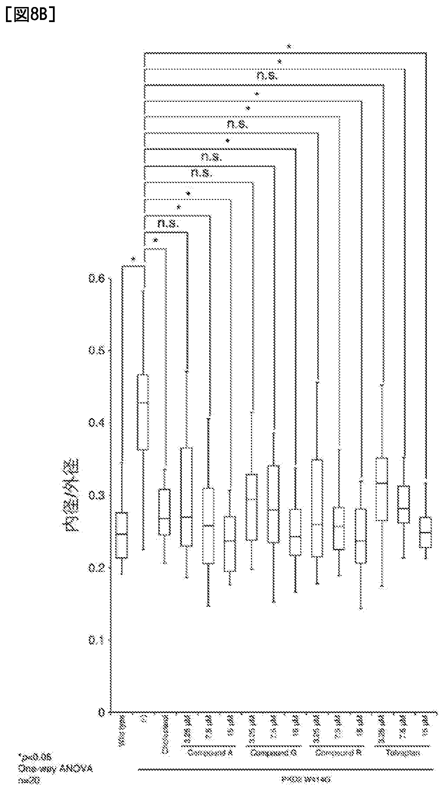

Using the PC2 mutant mIMCD3 cells, the compounds A, G, and R, as well as the existing drug tolvaptan, were each added at three concentrations of 3.75 µM, 7.5 µM, and 15 µM, and it was examined, in the same manner as in Example 4, whether each compound has an activity of suppressing enlargement of cyst tubule diameter. The results are shown in FIGS. 8A and 8B.

As shown in FIGS. 8A and 8B, it was confirmed that the compounds G and R have an activity of suppressing cyst size to a level comparable to that of tolvaptan, and the compound A has an activity of suppressing cyst size greater than that of tolvaptan.

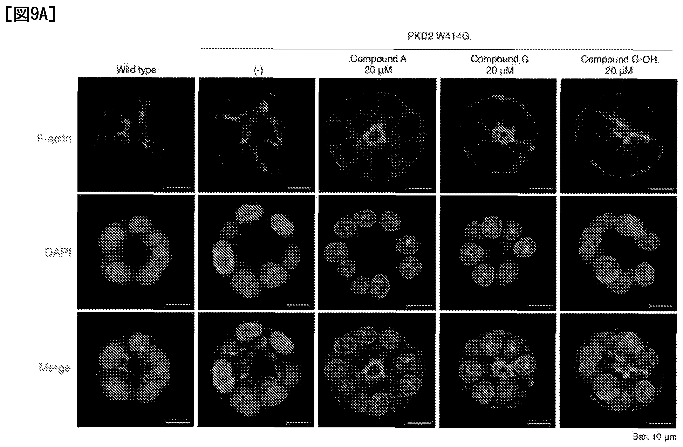

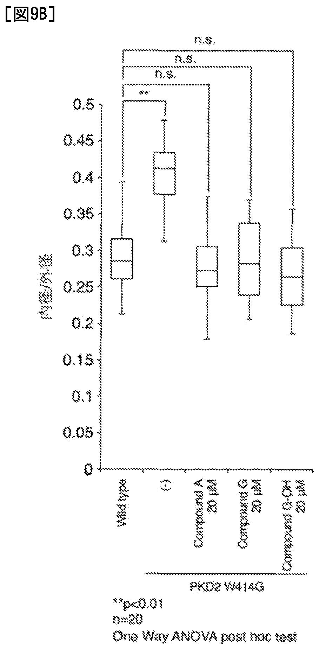

Further, using the PC2 mutant mIMCD3 cells, the compound A, the compound G, and the compound G-OH, which is a derivative of the compound G, were each added at 20 µM, and it was examined, in the same manner as in Example 4, whether each compound has an activity of suppressing enlargement of cyst tubule diameter. The results are shown in FIGS. 9A and 9B.

As shown in FIGS. 9A and 9B, it was confirmed that not only the compound G but also its derivative, the compound G-OH, has an activity of suppressing cyst size.

The compounds G and G-OH are shown below. The compound G corresponds to the above-mentioned compound (I-1), and the compound G-OH corresponds to the above-mentioned compound (I-6).

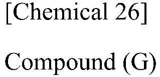

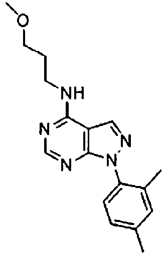

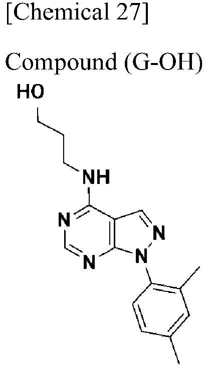

### [Example 6] Inhibitory effect on tubule diameter enlargement in cyst culture system reconstituting cyst formation via 3D culture of renal tubular cells: Compounds B, Q, K, J, and I

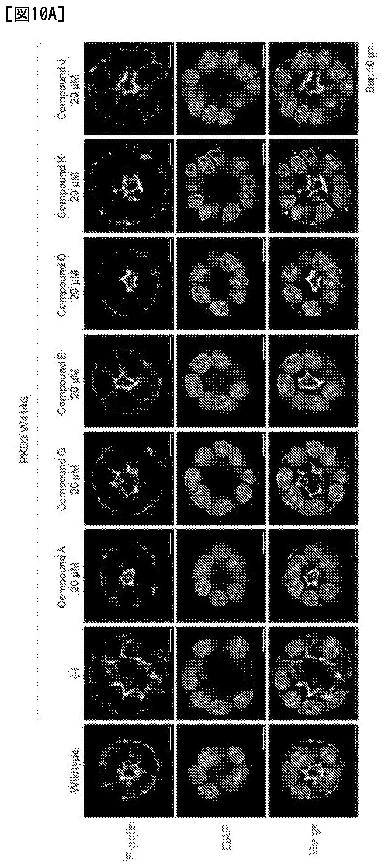

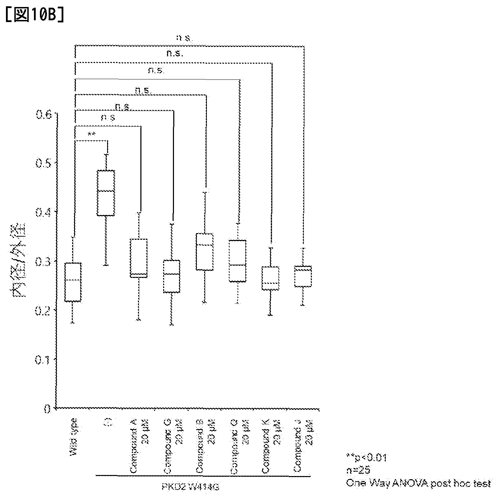

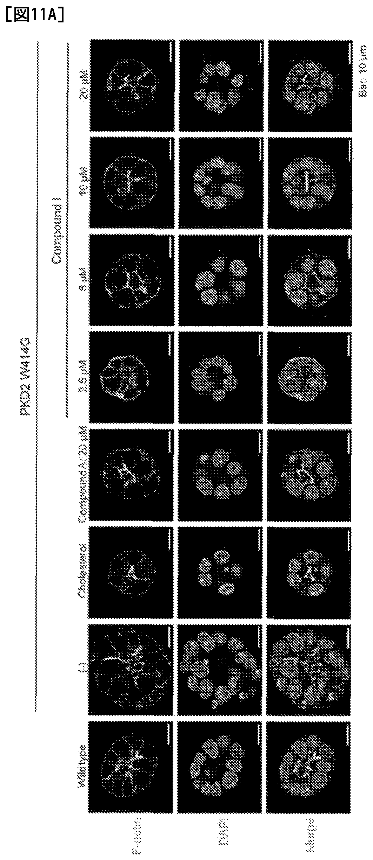

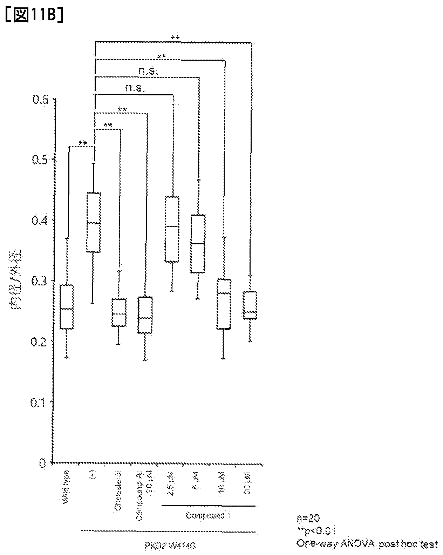

In Example 4, the compounds A, C, and R were used. Additionally, the compounds B, Q, K, J, and I were evaluated in the same manner for their inhibitory effects on tubule diameter enlargement. The above PC2 mutant mIMCD3 cells were used as cells, and the compounds A, G, B, Q, K, J, and I were used as compounds. The compounds A, G, B, Q, K, and J were each used at 20 µM, while the compound I was used at four concentrations of 2.5 µM, 5 µM, 10 µM, and 20 µM. In the same manner as in Example 4, images were obtained using a fluorescence microscope, and the inner diameter/outer diameter ratio was determined. The results for the compounds A, G, B, Q, K, and J are shown in FIGS. 10A and 10B, and the results for the compounds A and I are shown in FIGS. 11A and 11B.

As shown in FIGS. 10A and 10B, it was confirmed that the compounds B and Q have an activity of suppressing cyst size at a level comparable to that of the compound A, and the compounds K and J have an activity of suppressing cyst size at a level comparable to that of the compound G. Further, as shown in FIGS. 11A and 11B, it was confirmed that the compound I has also an activity of suppressing cyst size at a level comparable to that of the compound A when used at 10 µM and 20 µM.

The compounds B, Q, K, and J are shown below. The compound B corresponds to the above-mentioned compound (II-1), the compound Q corresponds to the above-mentioned compound (I-4), the compound K corresponds to the above-mentioned compound (I-3), and the compound J corresponds to the above-mentioned compound (I-2).

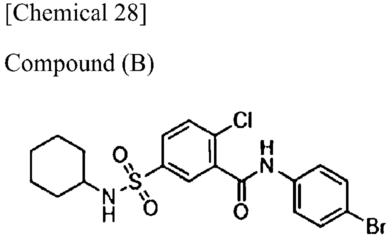

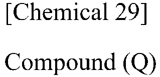

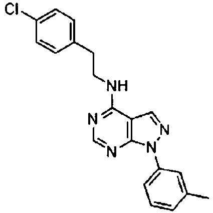

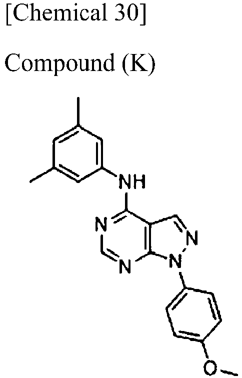

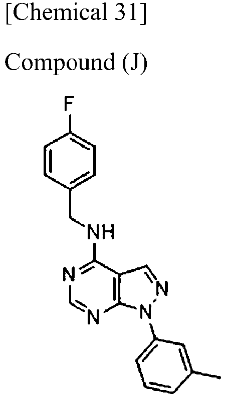

### [Example 7] Restoration effect of primary ciliary localization of polycystin 2 protein (PC2) in human induced pluripotent stem cells derived from patients with polycystic kidney disease: Comparison with tolvaptan

Using induced pluripotent stem cells derived from patients with autosomal dominant polycystic kidney disease (HPS2192 cells: RIKEN BRC RRID: CVCL_YS47 and HPS2636 cells: RIKEN BRC RRID: CVCL_YS58; RIKEN BioResource Research Center), it was examined whether the compound G-OH at 5 µM, 10 µM, and 20 µM, or the existing drug tolvaptan at 20 µM, has an activity of restoring localization of polycystin 2 protein to primary cilia.

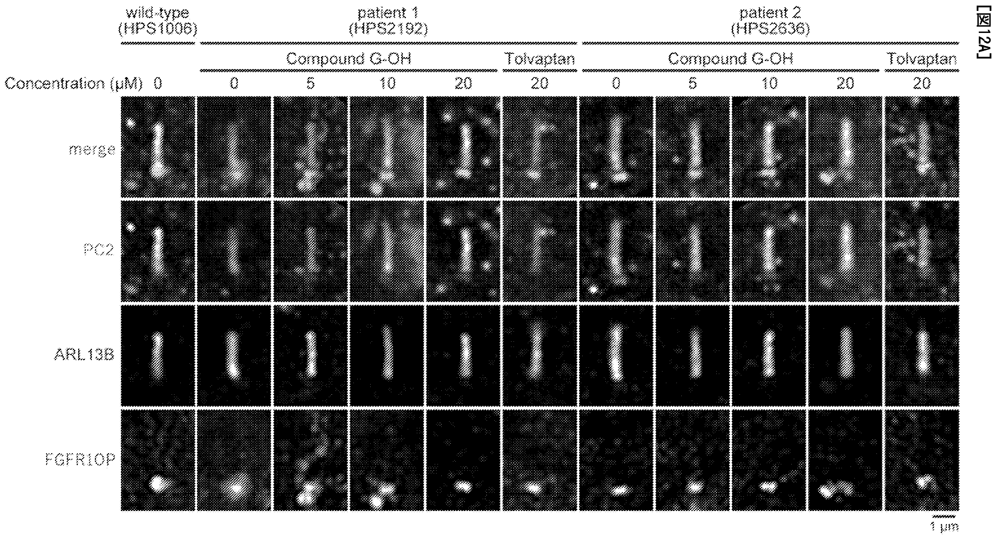

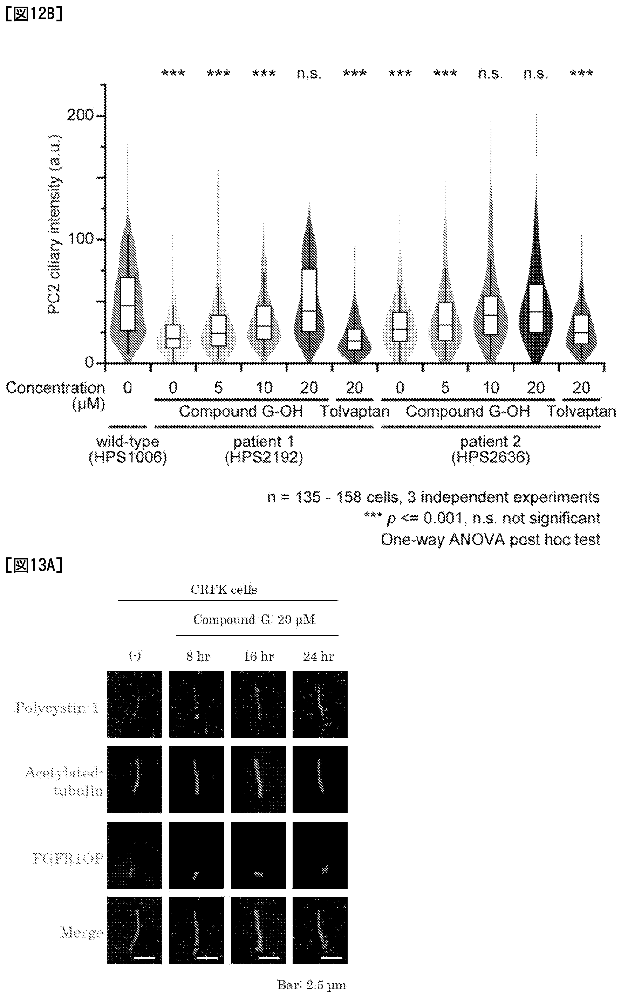

The above human induced pluripotent stem cells were treated with the compound G-OH or tolvaptan at 37°C for 24 hours, followed by multiplex staining with anti-polycystin 2 antibodies (Santa Cruz Biotechnology Inc.), anti-ARL13B antibodies (Santa Cruz Biotechnology Inc.) as a primary cilium marker, and anti-FGFR1OP antibodies (Proteintech Group, Inc.) as a centrosome marker. The results of observation of polycystin 2 in primary cilia using a confocal laser microscope and the results of quantification of polycystin 2 levels in primary cilia by immunostaining are shown in FIGS. 12A and 12B. As a control, induced pluripotent stem cells derived from a healthy individual (HPS1006 cells: RIKEN BRC RRID: CVCL_UP39; RIKEN BioResource Research Center) were used (wild-type).

As shown in FIGS. 12A and 12B, it was confirmed that the compound G-OH has an activity of restoring localization of polycystin 2 to primary cilia in human induced pluripotent stem cells derived from patients with polycystic kidney disease, even when polycystin 2 is mutated. Further, when the concentration dependency of the above restoration effect of the compound G-OH was examined, it was revealed by immunostaining that the amount of polycystin 2 in primary cilia increased in a dose-dependent manner. In contrast, the existing drug tolvaptan did not show activity of increasing the amount of polycystin 2 in primary cilia.

### [Example 8] Effect of increasing primary ciliary localization of polycystin 1 protein (PC1) in feline cells

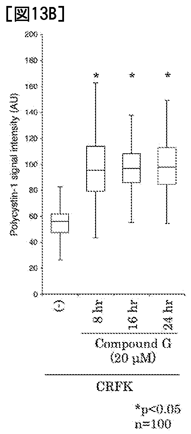

The effect of the compound G on increasing PC1 in primary cilia was evaluated. CRFK cells, which are a feline kidney-derived epithelial cell line, were prepared, and after treatment with the compound G (final concentration of 20 µM) at 37°C for 8, 16, or 24 hours, multiplex staining was performed with anti-polycystin 1 antibodies (Santa Cruz Biotechnology Inc.), anti-acetylated tubulin antibodies (Merck KGaA) as a primary cilium marker, and anti-FGFR1OP antibodies (Proteintech Group, Inc.) as a centrosome marker. The results of observation of PC1 in primary cilia using a confocal laser microscope and the results of measurement of fluorescence intensity of PC1 in primary cilia are shown in FIGS. 13A and 13B.

As shown in FIGS. 13A and 13B, it was confirmed that the compound G has an activity of increasing PC1 in primary cilia in CRFK cells, which are a feline kidney-derived epithelial cell line.

## Claims

1. A prophylactic or therapeutic agent for polycystic kidney disease, comprising, as an active ingredient, a compound having an activity of promoting an intermolecular interaction between oxysterol-binding protein-related protein 3 (ORP3) and vesicle-associated membrane protein-associated protein A (VAP-A) in a cell and having an activity of increasing cholesterol in primary cilia of a cell, or a pharmaceutically acceptable salt thereof.

2. The prophylactic or therapeutic agent for polycystic kidney disease according to claim 1, wherein the compound having the activity of promoting the intermolecular interaction and having the activity of increasing cholesterol in primary cilia of a cell is a compound including:
a 4-amino-1-phenylpyrazolopyrimidine skeleton;
a 5-(sulfamoyl)benzamide skeleton;
an N-thiazolylbenzamide skeleton; or
an N-benzylsuccinimide skeleton.

3. The prophylactic or therapeutic agent for polycystic kidney disease according to claim 1, wherein the compound having the activity of promoting the intermolecular interaction and having the activity of increasing cholesterol in primary cilia of a cell is a 4-amino-1-phenylpyrazolopyrimidine compound represented by a general formula (I): 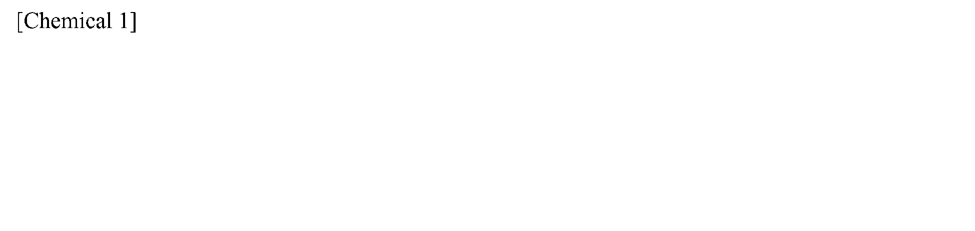 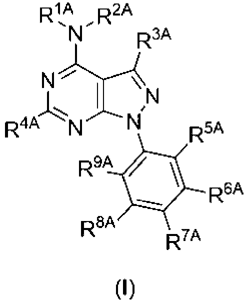
(wherein R^{1A} and R^{2A} are the same or different and each represent:
a hydrogen atom;
a C1-6 alkyl group optionally having a substituent;
a C1-6 alkoxy group optionally having a substituent;
a C2-6 alkenyl group optionally having a substituent;
a C2-6 alkynyl group optionally having a substituent; or
a phenyl group optionally having a substituent,
R^{3A} and R^{4A} are the same or different and each represent:
a hydrogen atom;
a halogen atom;
a nitro group;
a C1-6 alkyl group optionally having a substituent;
a C1-6 alkoxy group optionally having a substituent; or
a phenyl group optionally having a substituent, and
R^{5A}, R^{6A}, R^{7A}, R^{8A}, and R^{9A} are the same or different and each represent:
a hydrogen atom;
a halogen atom;
a nitro group;
a C1-6 alkyl group optionally having a substituent; or
a C1-6 alkoxy group optionally having a substituent), or a pharmaceutically acceptable salt thereof.

4. The prophylactic or therapeutic agent for polycystic kidney disease according to claim 1, wherein the compound having the activity of promoting the intermolecular interaction and having the activity of increasing cholesterol in primary cilia of a cell is:
a compound represented by a following formula (I-1) (4-methoxypropylamino-1-(2,4-dimethylphenyl)-1H-pyrazolo[3,4-d]pyrimidine);
a compound represented by a following formula (I-2) (4-(4-fluorobenzylamino)-1-(3-methylphenyl)-1H-pyrazolo[3,4-d]pyrimidine);
a compound represented by a following formula (I-3) (4-(3,5-dimethylphenyl)amino-1-(4-methoxyphenyl)-1H-pyrazolo[3,4-d]pyrimidine);
a compound represented by a following formula (I-4) (2-(4-chlorophenyl)ethylamino-1-(3-methylphenyl)-1H-pyrazolo[3,4-d]pyrimidine);
a compound represented by a following formula (I-5) (2-(1-cyclohexenyl)ethylamino-1-(3-methylphenyl)-1H-pyrazolo[3,4-d]pyrimidine); or
a compound represented by a following formula (I-6) (4-hydroxypropylamino-1-(2,4-dimethylphenyl)-1H-pyrazolo[3,4-d]pyrimidine).

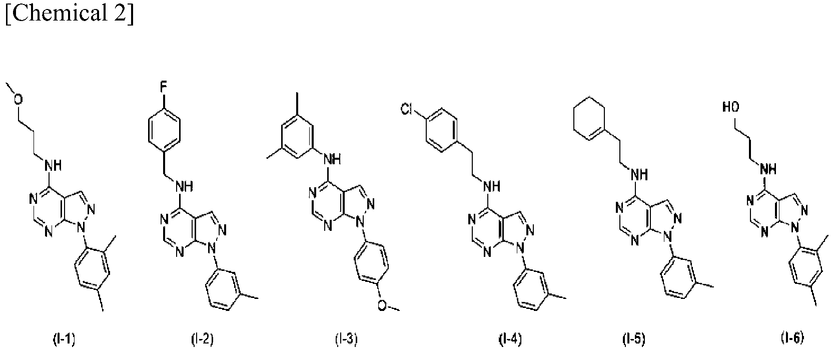

5. The prophylactic or therapeutic agent for polycystic kidney disease according to claim 1, wherein the compound having the activity of promoting the intermolecular interaction and having the activity of increasing cholesterol in primary cilia of a cell is a 5-(sulfamoyl)benzamide compound represented by a general formula (II). 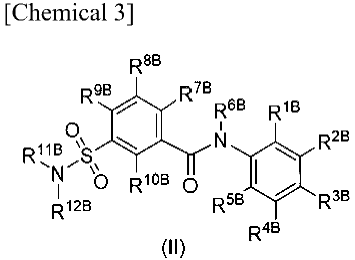
(wherein R^{1B}, R^{2B}, R^{3B}, R^{4B}, R^{5B}, R^{7B}, R^{8B}, R^{9B}, and R^{10B} are the same or different and each represent:
a hydrogen atom;
a halogen atom;
a nitro group;
a C1-6 alkyl group optionally having a substituent;
a C1-6 alkoxy group optionally having a substituent; or
a phenyl group optionally having a substituent,
R^{6B} represents:
a hydrogen atom; or
a C1-6 alkyl group optionally having a substituent, and
R^{11B} and R^{12B} are the same or different and each represent:
a hydrogen atom;
a C1-6 alkyl group optionally having a substituent;
a C1-6 alkoxy group optionally having a substituent;
a C3-8 cycloalkyl group optionally having a substituent;
a C3-6 cycloalkoxy group optionally having a substituent; or
a phenyl group optionally having a substituent)

6. The prophylactic or therapeutic agent for polycystic kidney disease according to claim 1, wherein the compound having the activity of promoting the intermolecular interaction and having the activity of increasing cholesterol in primary cilia of a cell is a compound represented by a following formula (II-1) or a compound represented by a following formula (II-2).

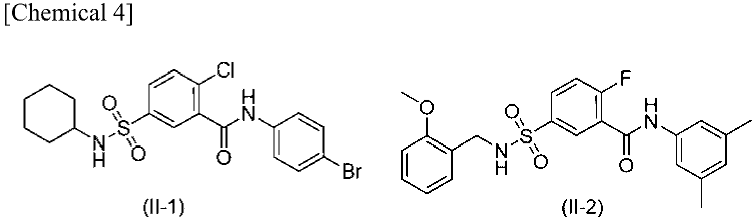

7. The prophylactic or therapeutic agent for polycystic kidney disease according to claim 1, wherein the compound having the activity of promoting the intermolecular interaction and having the activity of increasing cholesterol in primary cilia of a cell is an N-thiazolylbenzamide compound represented by a general formula (III). 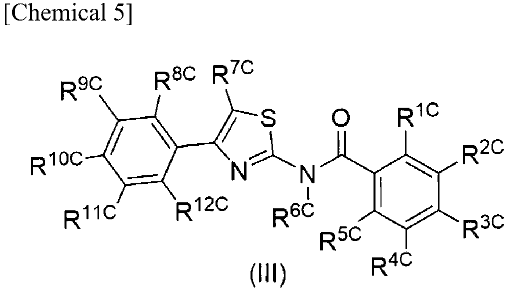
(wherein R^{1C}, R^{2C}, R^{3C}, R^{4C}, R^{5C}, R^{8C}, R^{9C}, R^{10C}, R^{11C}, and R^{12C} are the same or different and each represent:
a hydrogen atom;
a halogen atom;
a nitro group;
a C1-6 alkyl group optionally having a substituent;
a C1-6 alkoxy group optionally having a substituent; or
a phenyl group optionally having a substituent,
R^{6C} represents:
a hydrogen atom; or
a C1-6 alkyl group optionally having a substituent, and
R^{7C} represents:
a hydrogen atom;
a halogen atom;
a C1-6 alkyl group optionally having a substituent;
a C1-6 alkoxy group optionally having a substituent; or
a phenyl group optionally having a substituent)

8. The prophylactic or therapeutic agent for polycystic kidney disease according to claim 1, wherein the compound having the activity of promoting the intermolecular interaction and having the activity of increasing cholesterol in primary cilia of a cell is a compound represented by a following formula (III-1).

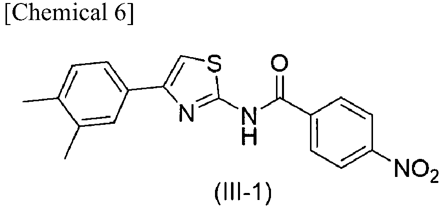

9. The prophylactic or therapeutic agent for polycystic kidney disease according to claim 1, wherein the compound having the activity of promoting the intermolecular interaction and having the activity of increasing cholesterol in primary cilia of a cell is an N-benzylsuccinimide compound represented by a general formula (IV). 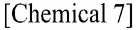 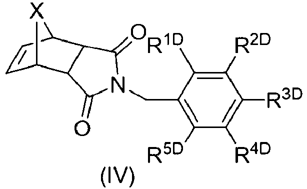
(wherein R^{1D}, R^{2D}, R^{3D}, R^{4D}, and R^{5D} are the same or different and each represent:
a hydrogen atom; or
a C1-6 alkyl group optionally having a substituent, and
X represents an oxygen atom or CH₂)

10. The prophylactic or therapeutic agent for polycystic kidney disease according to claim 1, wherein the compound having the activity of promoting the intermolecular interaction and having the activity of increasing cholesterol in primary cilia of a cell is a compound represented by a following formula (IV-1).

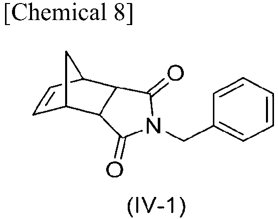

11. The prophylactic or therapeutic agent for polycystic kidney disease according to claim 1, for administration to a patient having a gene mutation in PKD1 and/or PKD2.
